(19) Europäisches Patentamt
European Patent Office
Office européen des brevets

(11) **EP 4 047 607 A1**

(12) **EUROPEAN PATENT APPLICATION**
published in accordance with Art. 153(4) EPC

(43) Date of publication:
**24.08.2022 Bulletin 2022/34**

(21) Application number: **20877483.6**

(22) Date of filing: **16.10.2020**

(51) International Patent Classification (IPC):
**G16B 40/20** (2019.01)  **A01K 67/027** (2006.01)
**C12Q 1/6809** (2018.01)  **G01N 33/15** (2006.01)
**G01N 33/50** (2006.01)

(52) Cooperative Patent Classification (CPC):
**A01K 67/027; C12Q 1/6809; G01N 33/15;
G01N 33/50; G16B 40/20**

(86) International application number:
**PCT/JP2020/039179**

(87) International publication number:
**WO 2021/075574 (22.04.2021 Gazette 2021/16)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **17.10.2019 JP 2019190332**

(71) Applicant: **Karydo Therapeutix, Inc.
Tokyo 102-0082 (JP)**

(72) Inventor: **SATO, Narutoku
Soraku-gun, Kyoto 619-0288 (JP)**

(74) Representative: **Vos, Derk
Maiwald Patentanwalts- und
Rechtsanwaltsgesellschaft mbH
Elisenhof
Elisenstraße 3
80335 München (DE)**

(54) **ARTIFICIAL INTELLIGENCE MODEL FOR PREDICTING INDICATIONS FOR TEST SUBSTANCES IN HUMANS**

(57)    An object is to predict an efficacy of a test substance even if the test substance has an efficacy that has not been known about the existing substances used to acquire training data.

An artificial intelligence model trained by a training method is used which includes inputting a first training data set, a second training data set and a third training data set in association with one another into an artificial intelligence model to train the artificial intelligence model. The first training data set is a set of data in which a set of data indicating the dynamics of a biomarker in one organ or each of multiple different organs collected from respective non-human animals to which multiple predetermined existing substances with a known indication in humans have been individually administered is linked with labels indicating respective names of the administered predetermined existing substances. The second training data set is a set of data in which labels indicating respective names of the multiple predetermined existing substances are linked with labels indicating the indications reported for each of the multiple predetermined existing substances. The third training data set is a set of data in which labels indicating the indications reported for each of the multiple predetermined existing substances are linked with information about adverse events reported correspondingly to each of these indications. The artificial intelligence model is for predicting an indication for a test substance in humans.

Fig.1

First training data

Second training data

Third training data

Multi-Organ Transcriptome of A

Multi-Organ Transcriptome of B

Multi-Organ Transcriptome of C

Drugs A

Drugs B

Drugs C

Indication 1

Indication 2

Indication 3

Indication 4

Drugs A

Drugs B

Drugs C

Indication 1

Indication 2

Indication 3

Indication 4

Adverse Event I

Adverse Event II

Adverse Event III

Adverse Event IV

Adverse Event V

Link-Prediction

Drug Candidate X

Multi-Organ Transcriptome

Prediction of target indication for drug candidate X

**Description**

Technical Field

This description discloses a method for predicting indications for a test substance in humans, a device for predicting indications for a test substance in humans, a program for predicting indications for a test substance in humans, and a method for training an artificial intelligence model for use in predicting indications for a test substance in humans and a trained artificial intelligence model.

Background Art

Development of a new drug starts with a drug discovery research (discovery phase) to find candidate substances for new drugs, followed by a preclinical trial (phase 0) using animals or cultured cells and phases I to III clinical trials in humans. Only the substances that have passed the trials are allowed to apply for permission to receive approval for manufacture and sale as pharmaceutical products from the Ministry of Health, Labour and Welfare. Then, even after having undergone a review required to be approved as a pharmaceutical product and having been launched on the market, there is provided a period for monitoring adverse events or effects that were not predicted during the development and approval review stages. As described above, it takes huge amount of time and money for one new drug to be launched on the market. On the other hand, the probability for a substance to proceed from the discovery phase to approval for manufacture and sale is said to be about 1.6%. It is also said that the only 13.8% of the substances that passed the preclinical trial exhibit an effect without adverse events during the clinical trials from the passage of the preclinical trial to the phase III and reach the application for approval. In other words, more than 80% of candidate substances drop out during the phase I to III clinical trials. The loss due to the dropout is said to be as enormous as 150 to 200 million dollars per substance.

As a method for assisting the exploration of candidate substances for new drugs in the development of a new drug, Patent Document 1 discloses a method including comparing test data of an organ-related index factor in each organ obtained from cells or tissues derived from one or more organs of individuals to which a test substance has been administered with preliminarily determined corresponding standard data of the organ-related index factor to obtain a pattern similarity for calculating the similarity of the pattern of the organ-related index factor, and predicting the efficacies or side effects of the test substance in the one or more organs and/or in organs other than the one or more organs using the pattern similarity of the organ-related index factor as an index.

Also, as a method for predicting efficacies or side effects of a candidate substance in the development of a new drug, Patent Document 2 discloses an artificial intelligence model for predicting one or multiple actions of a test substance on humans from the dynamics of transcriptome in multiple different organs which are the same as multiple different organs collected from non-human animals to which the test substance has been administered to prepare training data. The method includes inputting a data set indicating the dynamics of transcriptome in multiple different organs collected from non-human animals to which multiple existing substances with a known action on humans have been individually administered for each of the non-human animals and data indicating known actions of each existing substance on humans into the artificial intelligence model as training data to train the artificial intelligence model.

Citation List

Patent Document

Patent Document 1: WO2016/208776

Patent Document 2: Japanese Patent No. 6559850

Summary of Invention

Technical Problem

One object of this disclosure is to predict effectively an indication for a test substance in humans from the dynamics of a biomarker in response to the administration of the test substance to animals other than humans.

By the method described in Patent Document 2, it is only possible to predict efficacies already known about the existing substances used to acquire transcriptome data of multiple organs as training data.

An object of the present invention is to predict an efficacy of a test substance even if the test substance has an efficacy that has not been known about existing substances used to acquire training data.

Solution to Problem

The present invention may include the following aspects as embodiments.

Embodiment 1. A certain embodiment of the present invention relates to a method for training an artificial intelligence model. The training method includes inputting a first training data set, a second training data set and a third training data set in association with one another into an artificial intelligence model to train the artificial intelligence model, the first training data set being a set of data in which a set of data indicating the dynamics of a biomarker in one organ or each of multiple different organs collected from respective non-human animals to which multiple predetermined existing substances with a known indication in humans have been individually administered is linked with labels indicating respective names of the administered predetermined existing substances, the second training data set being a set of data in which labels indicating respective names of the multiple predetermined existing substances are linked with labels indicating the indications reported for each of the multiple predetermined existing substances, the third training data set being a set of data in which labels indicating the indications reported for each of the multiple predetermined existing substances are linked with information about adverse events reported correspondingly to each of these indications, wherein the artificial intelligence model is for predicting an indication for a test substance in humans.

Embodiment 2. In the training according to Embodiment 1, the first training data set and the third training data set are linked by means of the second training data set to generate a fourth training data set, and the fourth training data set is input into the artificial intelligence model.

Embodiment 3. In the training method according to Embodiment 1 or 2, the information about adverse events includes labels indicating the adverse events, and the presence or absence or frequencies of occurrence of the adverse events in the indications.

Embodiment 4. In the training method according to any one of Embodiments 1 to 3, the biomarker is a transcriptome.

Embodiment 5. In the training method according to any one of Embodiments 1 to 4, the artificial intelligence model is a One-Class SVM

Embodiment 6. A certain embodiment of the present invention relates to a training device for an artificial intelligence model. The training device includes a processing part, wherein the processing part inputs a first training data set, a second training data set and a third training data set in association with one another into an artificial intelligence model to train the artificial intelligence model, the first training data set being a set of data in which a set of data indicating the dynamics of a biomarker in one organ or each of multiple different organs collected from respective non-human animals to which multiple predetermined existing substances with a known indication in humans have been individually administered is linked with labels indicating respective names of the administered predetermined existing substances, the second training data set being a set of data in which labels indicating respective names of the multiple predetermined existing substances are linked with labels indicating the indications reported for each of the multiple predetermined existing substances, the third training data set being a set of data in which labels indicating the indications reported for each of the multiple predetermined existing substances are linked with information about adverse events reported correspondingly to each of these indications, and wherein the artificial intelligence model is for predicting an indication for a test substance in humans.

Embodiment 7. A certain embodiment of the present invention relates to a program for training an artificial intelligence model that, when executed by a computer, causes the computer to execute the step of inputting a first training data set, a second training data set and a third training data set in association with one another into an artificial intelligence model to train the artificial intelligence model. In the program, the first training data set is a set of data in which a set of data indicating the dynamics of a biomarker in one organ or each of multiple different organs collected from respective non-human animals to which multiple predetermined existing substances with a known indication in humans have been individually administered is linked with labels indicating respective names of the administered predetermined existing substances, the second training data set is a set of data in which labels indicating respective names of the multiple predetermined existing substances are linked with labels indicating the indications reported for each of the multiple predetermined existing substances, and the third training data set is a set of data in which labels indicating the indications reported for each of the multiple predetermined existing substances are linked with information about adverse events reported correspondingly to each of these indications, wherein the artificial intelligence model is for predicting indication for a test substance in humans.

Embodiment 8. A certain embodiment of the present invention relates to a method for predicting an indication for a test substance in humans. The method includes the steps of: acquiring a first test data set, the first test data set being a set of data indicating the dynamics of a biomarker in one or multiple organs collected from non-human animals to which a test substance has been administered, and inputting the first test data set and a second test data set into an artificial intelligence model trained by a method according to any one of Embodiments 1 to 5 to use the trained artificial intelligence model to predict an indication for the test substance in humans based on the first test data set and the second test data set input thereinto, the second test data set being a set of data in which labels of multiple known indications are linked with information about adverse events reported correspondingly to each of the multiple known indications.

Embodiment 9. In the prediction method according to Embodiment 8, the test substance does not include an existing substance or an equivalent substance of an existing substance.

Embodiment 10. In the prediction method according to Embodiment 8 or 9, the test substance is one selected from existing substances or equivalent substances of existing substances.

Embodiment 11. A certain embodiment of the present invention relates to a prediction device for predicting an indication for a test substance in humans. The prediction device includes a processing part, wherein the processing part inputs a first test data set and a second test data set into an artificial intelligence model trained by a method according to any one of Embodiments 1 to 5 to use the trained artificial intelligence model to predict an indication for the test substance in humans based on the first test data set and the second test data set input thereinto, the first test data set being a set of data indicating the dynamics of a biomarker in one or multiple organs corresponding to one or multiple organs collected from non-human animals to which the test substance has been administered to generate the first training data set, the second test data set being a set of data in which labels of multiple known indications are linked with information, acquired to generate a third training data set, about adverse events reported correspondingly to each of the multiple known indications.

Embodiment 12. A certain embodiment of the present invention relates to a computer program for predicting an indication for a test substance in humans that, when executed by a computer, causes the computer to execute the step of: inputting a first test data set and a second test data set into an artificial intelligence model trained by a method according to any one of Embodiments 1 to 5 to use the trained artificial intelligence model to predict an indication for the test substance in humans based on the first test data set and the second test data set input thereinto, the first test data set being a set of data indicating the dynamics of a biomarker in one or multiple organs corresponding to one or multiple organs collected from non-human animals to which the test substance has been administered to generate the first training data set, the second test data set being a set of data in which labels of multiple known indications are linked with information about adverse events reported correspondingly to each of the multiple known indications.

Embodiment 13. A certain embodiment of the present invention relates to a prediction system for predicting an indication for a test substance in humans. The system includes: a server device for transmitting a first test data set, the first test data set being a set of data indicating the dynamics of a biomarker in one or multiple organs collected from non-human animals to which the test substance has been administered, and a prediction device for predicting an action of the test substance on humans connected to the server device via a network. The server device includes a communication part for transmitting the first test data set, the prediction device includes a processing part and a communication part, wherein the processing part acquires the first test data set transmitted via the communication part of the server device via the communication part of the prediction device, and inputs the acquired first test data set and a second test data set into an artificial intelligence model trained by a method according to any one of Embodiments 1 to 5 to use the trained artificial intelligence model to predict an indication for the test substance in humans based on the first test data set and the second test data set input thereinto, the first test data set being a set of data indicating the dynamics of a biomarker in one or multiple organs corresponding to one or multiple organs collected from non-human animals to which the test substance has been administered to generate the first training data set, the second test data set being a set of data in which labels of multiple known indications are linked with information, acquired to generate a third training data set, about adverse events reported correspondingly to each of the multiple known indications.

Embodiment 14. A certain embodiment of the present invention relates to a method for using a first training data set, a second training data set and a third training data set to train an artificial intelligence model for predicting an indication for a test substance in humans, the first training data set being a set of data in which a set of data indicating the dynamics of a biomarker in one organ or each of multiple different organs collected from respective non-human animals to which multiple predetermined existing substances with a known indication in humans have been individually administered is linked with labels indicating the names of existing substances administered to acquire the set of data indicating the dynamics of a biomarker, the second training data set being a set of data in which labels indicating respective names of the multiple predetermined existing substances are linked with labels indicating the indications reported for each of the multiple predetermined existing substances, the third training data set being a set of data in which labels indicating the indications are linked with information about adverse events reported correspondingly to each of the indications.

Embodiment 15. This embodiment relates to a method for using a first test data set and a second test data set as test data for predicting an indication for a test substance in humans. In the method, the first test data set is a set of data indicating the dynamics of a biomarker in one or multiple organs corresponding to one or multiple organs collected from non-human animals to which the test substance has been administered to generate the first training data set, and the second test data set is a set of data in which labels of multiple known indications are linked with information about adverse events reported correspondingly to each of the multiple known indications.

Advantageous Effects of Invention

It is possible to predict an efficacy of a test substance even if the test substance has an efficacy that has not been known about existing substances used to acquire training data.

Brief Description of Drawings

FIG. 1 illustrates an overview of the present invention.

FIG. 2 illustrates an overview of the invention described in Patent Document 2 (prior art).

FIG. 3 shows examples of training data. FIG. 3(A) shows examples of first training data. FIG. 3(B) shows examples of second training data. FIG. 3(C) shows examples of third training data. FIG. 3(D) shows examples of fourth training data.

FIG. 4(A) illustrates a hardware configuration of a training system. FIG. 4(B) illustrates a hardware configuration of a prediction system.

FIG. 5 illustrates a hardware configuration of a training device.

FIG. 6 is a flowchart showing the flow of processing by a training program.

FIG. 7 illustrates a hardware configuration of a prediction device.

FIG. 8 is a flowchart showing the flow of processing by a prediction program.

FIG. 9 illustrates a hardware configuration of a server device.

FIG. 10 is a flowchart showing the flow of processing in a prediction system.

FIG. 11 shows a prediction result from an artificial intelligence trained without using transcriptome data of test drugs.

FIG. 12 shows a prediction result from an artificial intelligence trained using transcriptome data of test drugs.

FIG. 13 shows some of decision function values of alendronate.

Description of Embodiments

1. Overview of training method and prediction method, and description of terms

First, a method for training an artificial intelligence and a prediction method as certain embodiments of this disclosure are outlined. Also, the differences between conventional methods and the training and prediction methods included in this disclosure are described.

The prediction method predicts an indication for a test substance in humans. Preferably, the prediction method predicts an indication for a test substance in humans based on information about the dynamics of a biomarker in non-human animals to which an existing substance with a known action on humans has been administered, known indications, and adverse events reported correspondingly to the known indications. The prediction is made using an artificial intelligence model.

(1) Training phase

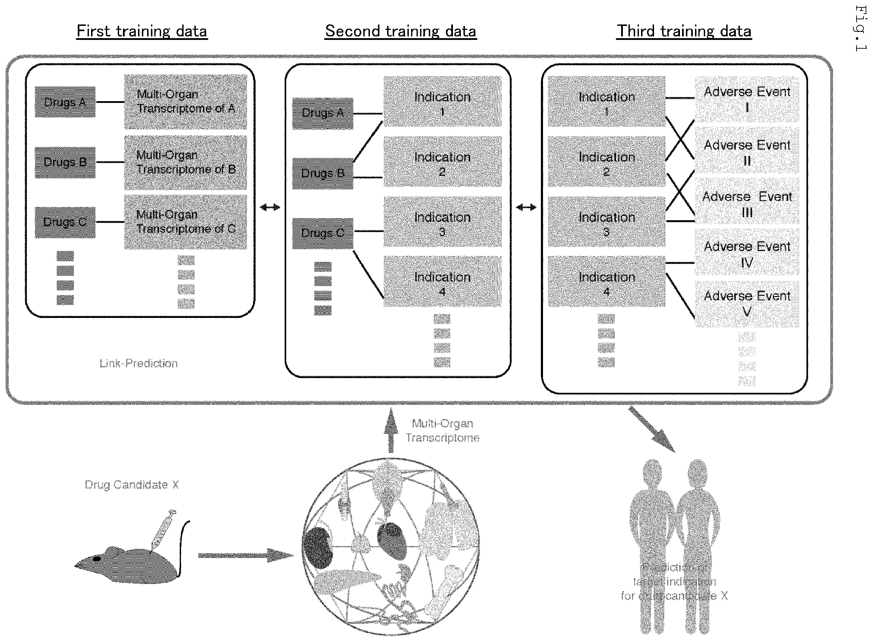

As shown in FIG. 1, the artificial intelligence model used for the prediction is preferably trained by means of a data set including three types of training data sets, i.e., a first training data set, a second training data set and a third training data set, which are associated with one another.

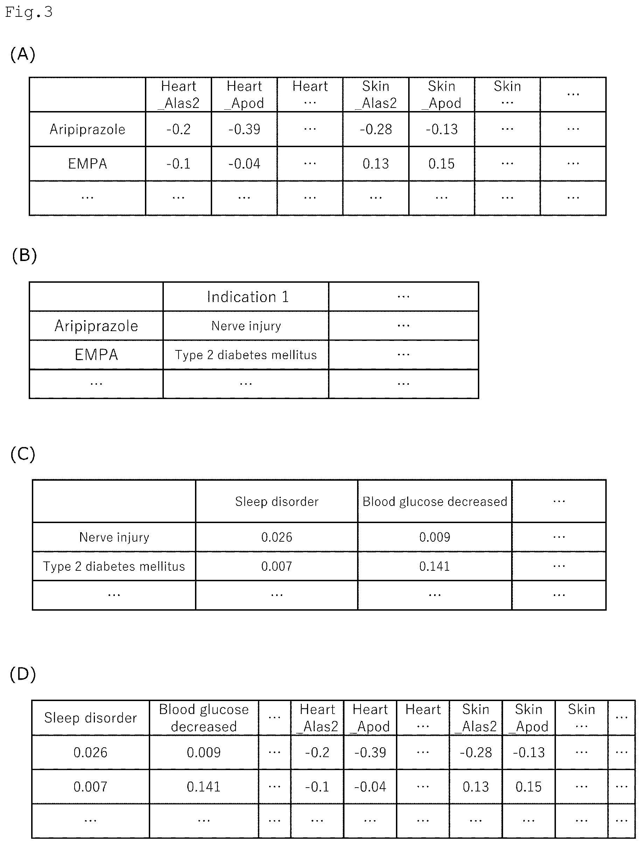

As shown in FIG. 1, the first training data set is a set of data in which a set of data indicating the dynamics of a biomarker in one organ or each of multiple different organs collected from respective non-human animals after multiple predetermined existing substances with a known indication in humans have been individually administered to the non-human animals is linked with labels indicating respective names of the administered predetermined existing substances. The first training data set is generated as shown in FIG. 1. For example, Drugs A, B and C as predetermined existing substances are individually administered to non-human animals such as mice and organs or tissues as parts of organs are respectively collected from the non-human animals. Next, the dynamics of a biomarker in the collected organs or tissues is analyzed and a first training data set is generated from [data indicating respective organ names and the dynamics of a biomarker] and [respective names of the administered drugs]. FIG. 3(A) shows a more specific example of the first training data set. In the example of the first training data set shown in FIG. 3(A), the leftmost column is referred to as "first column." In the first column shown in FIG. 3(A), a drug name "Aripiprazole" and a drug name "EMPA" are shown as examples. In the second and subsequent columns, the expression level of RNA in each organ is shown. "Heart" and "Skin" are labels of organ names, and "Alas2" and "Apod" are labels of names of genes whose expression was analyzed. In the second and subsequent columns and the second and subsequent rows, values indicating expression levels of respective genes have been entered as elements. In the first training data set, [labels indicating organ names and labels indicating gene names] and [values indicating expression levels of respective genes] correspond to labels indicating drug names.

As shown in FIG. 1, the second training data set are a set of data in which labels indicating respective names of the multiple predetermined existing substances administered to obtain the first training data set (the first column of FIG. 3(A)) are linked with labels indicating the indications reported for each of the multiple predetermined existing substances. FIG. 3(B) shows a specific example of the second training data set. In the example of the second training data set shown in FIG. 3(B), the leftmost column is referred to as "first column." In the first column shown in FIG. 3(B), a drug name "Aripiprazole" and a drug name "EMPA" are shown as examples. The second and subsequent columns show indications reported for each drug listed in the first column. Here, "Nerve injury" is shown as a name label indicating an indication for the drug name "Aripiprazole," and "Type 2 diabetes mellitus" is shown as a name label indicating an indication for the drug name "EMPA."

As shown in FIG. 1, the third training data is a set of data in which labels indicating the indications reported for each of the multiple predetermined existing substances administered to obtain the first training data set as shown in FIG. 3(B) are linked with information about adverse events reported correspondingly to each of these indications. Here, the information about adverse events may include labels indicating the names of adverse events, and the presence or absence or frequencies of occurrence of adverse events. FIG. 3(C) shows a more specific example of the third training data set. In the example of the third training data set shown in FIG. 3(C), the leftmost column is referred to as "first column." "Nerve injury," which is an indication for the drug name "Aripiprazole" shown in "Indication 1" of FIG. 3(B), is shown in the first column of FIG. 3(C). Also, as an indication for the drug name "EMPA" shown in "Indication 1" of FIG. 3(B), "Type 2 diabetes mellitus" is shown in the first column of FIG. 3(C). The uppermost column of the second and subsequent columns of FIG. 3(C) shows labels indicating the names of adverse events, and "Sleep disorder" and "Blood glucose decreased" are shown here. The numerical values in the second and subsequent rows of the second column of FIG. 3(C) show the frequencies of occurrence of respective adverse events.

In the training method of this embodiment, it is a fourth training data set that is input into an artificial intelligence, the fourth training data set being generated by linking the first training data set with the third training data set by means of the second training data set.

FIG. 3(D) shows an example of the fourth training data set. In the example of the fourth training data set shown in FIG. 3(D), the leftmost column is referred to as "first column." In the first and second columns, labels indicating the names of the adverse events shown in FIG. 3(C) and the frequencies of occurrence of respective adverse events are shown. Also, in the fourth and subsequent columns, labels indicating the names of organs and labels of the names of genes, and the expression levels of the genes shown in FIG. 3(A) are shown. In other words, FIG. 3(D) shows a data set including the frequencies of occurrence of the adverse events in the second and subsequent columns of FIG. 3(C) which are substituted for the labels in the first column showing the names of drugs in FIG. 3(A).

(2) Prediction phase

An artificial intelligence model trained as described in Section 1.(1) above is used to predict an indication for a test substance in humans. The test data sets that are input into the trained artificial intelligence model to predict an indication are a first test data set and a second test data set. The first test data set is input into the trained artificial intelligence model together with the second test data set.

The first test data set is a set of data indicating the dynamics of a biomarker in one or multiple organs collected from non-human animals to which the test substance has been administered. Also, the multiple organs correspond to the organs collected to generate the first training data set. Preferably, the first test data is data in which [labels indicating organ names and labels indicating gene names] are linked with [values indicating the expression levels of respective genes] which is obtained by administering one test substance to non-human animals and analyzing transcriptome in one or multiple organs collected therefrom.

The second test data set is a set of data in which labels of multiple known indications are linked with the information, acquired to generate a third training data set, about adverse events reported correspondingly to each of the multiple known indications. Here, the multiple known indications may include not only the indications used as the second training data but also known indications registered in an external database. In the known indications, the term "multiple" may be intended to mean, for example, 100, 500, 1000 or 2000, or more.

Here, in the prediction method, the test substance does not have to be an existing substance or an equivalent substance of an existing substance. When the test substance is not an existing substance or an equivalent substance of an existing substance, the prediction method serves as a method for predicting an indication for a new substance.

Also, in the prediction method, an existing substance or an equivalent substance of an existing substance may be included as a test substance. In this case, the prediction method serves as a drug repositioning method for exploring a new indication for an existing substance or an equivalent substance of an existing substance. When the prediction method described in this specification is used as a drug repositioning method, it is preferred to include the test substance in the existing substances administered to acquire the first training data set. In this way, the prediction accuracy can be increased.

(3) Comparison with conventional method

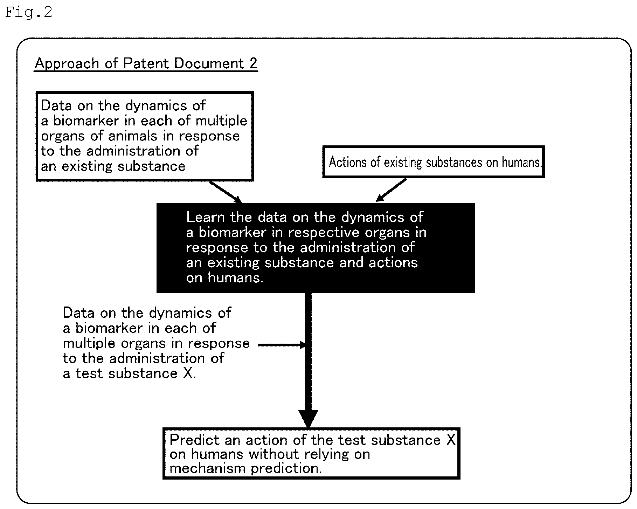

The conventional method shown in FIG. 2 is a method described in Patent Document 2, in which Drugs A, B and C as existing substances, for example, are individually administered to non-human animals such as mice and organs or tissues as parts of organs are collected from the respective non-human animals. Next, the dynamics of a biomarker in the collected organs or tissues is analyzed to generate a first training data set. Also, second training data is generated from a human clinical database for, for example, adverse events, indications, drug kinetics and indications for existing substances. Then, the artificial intelligence model shown in FIG. 2 is generated by training using the first training data set and the second training data. In other words, in the conventional method, an artificial intelligence model is constructed by associating the dynamics of a biomarker with each one of adverse events, indications, drug kinetics or indications for existing substances. Also, the test data used in the conventional method is data indicating the dynamics of a biomarker in one organ or multiple different organs corresponding to one organ or multiple organs collected from non-human animals to which the test substance has been administered to generate the first training data set.

This embodiment is different from the conventional method in that not only the dynamics of a biomarker but also information about adverse events that is substituted for the indication names are used as training data. Also, as test data as well, not only the dynamics of a biomarker but also information about multiple known indications and adverse events are used.

It is, therefore, possible to predict an indication for a test substance even if the test substance has an indication that has not been known about existing substances used to acquire the training data.

(4) Description of terms

In this disclosure, the non-human animals are not limited. Examples include mammals such as mice, rats, dogs, cats, rabbits, cows, horses, goats, sheep and pigs, and birds such as chickens. Preferably, the non-human animals are mammals such as mice, rats, dogs, cats, cows, horses and pigs, more preferably mice, rats or the like, and still more preferably mice. The non-human animals also include fetuses, chicks and so on of the animals.

In this disclosure, the term "substance" may include, for example, compounds; nucleic acids; carbohydrates; lipids; glycoproteins; glycolipids; lipoproteins; amino acids; peptides; proteins; polyphenols; chemokines; at least one metabolic substance selected from the group consisting of ultimate metabolites, intermediary metabolites and synthetic raw material substances of the above-mentioned substances; metal ions; or microorganisms. Also, the substance may be a simple substance or may be a mixture of multiple substances. Preferably, the term "substance" includes, for example, pharmaceutical products, quasi-pharmaceutical products, cosmeceutical products, foods, foods for specified health use, foods with functional claims, and candidates therefor. Also, the term "substance" may also include substances whose testing was discontinued or suspended during a preclinical or clinical trial for pharmaceutical approval.

The "existing substance" is not limited as long as it is an existing substance. Preferably, it is a substance with a known action on humans. Also, the term "equivalent substance of an existing substance" may include those that are similar in structure to an existing substance and has a similar action thereto. The term "similar action" here is intended to mean having the same kind of action as an existing substance although the intensity of the action may be the same or different.

The "adverse event" is not limited as long as it is an action that is determined to be harmful to humans. Preferred examples include adverse events listed in an external database such as FAERS (https://www.fda.gov/Drugs/GuidanceComplianceRegulatoryInformation/Surveillance/Adverse DrugEffects/ucm082193.htm) or clinicaltrials.gov (https://clinicaltrials.gov/).

The "indication" is not limited as long as it is a disorder or symptom in humans that should be mitigated, treated, arrested or prevented. Examples of the disorder or symptom include disorders or symptoms listed in an external database such as the above-mentioned FAERS, all drug labels of DAILYMED (https://dailymed.nlm.nih.gov/dailymed/spl-resources-all-drug-labels.cfm ), Medical Subject Headings (https://www.nlm.nih.gov/mesh/meshhome.html), Drugs@FDA (https://www.accessdata.fda.gov/scripts/cder/daf/), or International Classification of Diseases (https://www.who.int/health-topics/international-classification-of-diseases). More specifically, examples of the indication include ischemic diseases such as thrombosis, embolism and stenosis (in particular, heart, brain, lungs, large intestine, etc.); circulatory disorders such as aneurysm, phlebeurysm, congestion and hemorrhage (aortae, veins, lungs, liver, spleen , retinae, etc.); allergic diseases such as allergic bronchitis and glomerulonephritis; dementia such as Alzheimer's dementia; degenerative disorders such as Parkinson's disease, amyotrophic lateral sclerosis and myasthenia gravis (nerves, skeletal muscles, etc.); tumors (benign epithelial tumor, benign non-epithelial tumor, malignant epithelial tumor, malignant non-epithelial tumor); metabolic diseases (abnormal carbohydrate metabolism, abnormal lipid metabolism, electrolyte imbalance); infectious diseases (bacteria, viruses, rickettsia, chlamydia, fungi, protozoa, parasite, etc.); and symptoms or illnesses associated with autoimmune diseases or the like such as renal diseases, systemic erythematodes and multiple sclerosis.

The incidence rate of an adverse event can be obtained by the following method. A word indicating the name of the adverse event is extracted by, for example, text extraction from a database such as clinicaltrials.gov, FAERS, or all drug labels of DAILYMED as described above. One extracted word can be counted as one reported adverse event. For one existing substance, the incidence rate can be obtained according to the equation: Incidence rate = (the number of cases reported for one adverse event)/(the total number of cases of adverse events reported for the existing substance). When explanation related to actions is registered in text form in a database, syntactic analysis, word segmentation, semantic analysis or the like may be performed on the registered texts by natural language processing before the extraction of the texts corresponding to the actions.

The "organ" is not limited as long as it is an organ present in the body of a mammal or bird as described above. For example, in the case of a mammal, the organ is at least one selected from circulatory system organs (heart, artery, vein, lymph duct, etc.), respiratory system organs (nasal cavity, paranasal sinus, larynx, trachea, bronchi, lung, etc.), gastrointestinal system organs (lip, cheek, palate, tooth, gum, tongue, salivary gland, pharynx, esophagus, stomach, duodenum, jejunum, ileum, cecum, appendix, ascending colon, transverse colon, sigmoid colon, rectum, anus, liver, gallbladder, bile duct, biliary tract, pancreas, pancreatic duct, etc.), urinary system organs (urethra, bladder, ureter, kidney), nervous system organs (cerebrum, cerebellum, mesencephalon, brain stem, spinal cord, peripheral nerve, autonomic nerve, etc.), female reproductive system organs (ovary, oviduct, uterus, vagina, etc.), breast, male reproductive system organs (penis, prostate, testicle, epididymis, vas deferens), endocrine system organs (hypothalamus, pituitary gland, pineal body, thyroid gland, parathyroid gland, adrenal gland, etc.), integumentary system organs (skin, hair, nail, etc.), hematopoietic system organs (blood, bone marrow, spleen, etc.), immune system organs (lymph node, tonsil, thymus, etc.), bone and soft tissue organs (bone, cartilage, skeletal muscle, connective tissue, ligament, tendon, diaphragm, peritoneum, pleura, adipose tissue (brown adipose, white adipose), etc.), and sensory system organs (eyeball, palpebra, lacrimal gland, external ear, middle ear, inner ear, cochlea, etc.). Preferably, the "organ" can be at least one selected from bone marrow, pancreas, skull bone, liver, skin, brain, pituitary gland, adrenal gland, thyroid gland, spleen, thymus, heart, lung, aorta, skeletal muscle, testicle, epididymal fat, eyeball, ileum, stomach, jejunum, large intestine, kidney, and parotid gland. Preferably, all of bone marrow, pancreas, skull bone, liver, skin, brain, pituitary gland, adrenal gland, thyroid gland, spleen, thymus, heart, lung, aorta, skeletal muscle, testicle, epididymal fat, eyeball, ileum, stomach, jejunum, large intestine, kidney, and parotid gland are used in the prediction according to this disclosure. The term "multiple organs" is not limited as long as the number of organs is two or more. For example, the multiple organs can be selected from 2, 3, 4, 5, 6, 7, 8, 9, 10, 11, 12, 13, 14, 15, 16, 17, 18, 19, 20, 21, 22, 23, or 24 types of organs.

The term "organ-derived" is intended to mean, for example, being collected from an organ, or being cultured from cells, tissues or a body fluid of a collected organ.

The term "body fluid" includes, for example, serum, plasma, urine, spinal fluid, ascites, pleural effusion, saliva, gastric juice, pancreatic juice, bile, milk, lymph and intercellular fluid.

The term "biomarker" means a biological substance that can be varied in the cells or tissues of each organ and/or in a body fluid depending on the administration of the substance. An example of a biological substance that may serve as a "biomarker," is at least one selected from nucleic acids; carbohydrates; lipids; glycoproteins; glycolipids; lipoproteins; amino acids, peptides; proteins; polyphenols; chemokines; at least one metabolic substance selected from the group consisting of ultimate metabolites, intermediary metabolites and synthetic raw material substances of the above-mentioned substances; metal ions and so on. More preferred is a nucleic acid. The biomarker is preferably a group of biological substances that are varied in the cells or tissues of each organ and/or in a body fluid depending on the administration of the substance. An example of a group of biological substances can be a group of at least one kind selected from nucleic acids; carbohydrates; lipids; glycoproteins; glycolipids; lipoproteins; amino acids, peptides; proteins; polyphenols; chemokines; at least one metabolic substance selected from the group consisting of ultimate metabolites, intermediary metabolites and synthetic raw material substances of the above-mentioned substances; metal ions and so on.

The term "nucleic acids" preferably means a group of RNAs contained in transcriptome, such as mRNAs, non-coding RNAs and microRNAs, more preferably a group of mRNAs. The RNAs are preferably mRNAs, non-coding RNAs and/or microRNAs that may be expressed in the cells or tissues of the above organs or cells in a body fluid, more preferably mRNAs, non-coding RNAs and/or microRNAs that may be detected by RNA-Seq or the like (https://www.ncbi.nlm.nih.gov/gene?LinkName=genome_gene&from_uid=52, http://jp.support.illumina.com/sequencing/sequencing_software/igenome.html). Preferably, all RNAs that can be analyzed by RNA-Seq are used for the prediction according to this disclosure.

The term "set of data indicating the dynamics of a biomarker" is intended to mean a set of data indicating that the biomarker has or has not been varied in response to the administration of an existing substance. Preferably, the dynamics of a biomarker indicates that the biomarker has been varied in response to the administration of an existing substance. The data can be acquired by, for example, the following method. For tissues, cells or body fluids derived from certain organs collected from non-human animals to which an existing substance has been administered, the abundance or concentration of each biomarker is measured to acquire a measurement value for each organ of the individuals to which the existing substance has been administered. Also, from non-human animals to which the existing substance has not been administered, the abundance or concentration of each biomarker is measured for tissues, cells or body fluids derived from organs corresponding to the organs from which measurement values of the individuals to which the existing substance has been administered were acquired in the same manner to acquire measurement values in non-administered individuals. The measurement values of each biomarker derived from each organ of the individuals to which the existing substance has been administered are compared with the measurement values in non-administered individuals of the biomarker for each organ corresponding to the biomarkers in the individuals to which the existing substance has been administered to acquire values indicating the differences therebetween as data. Here, the term "corresponding to" means that the organs and biomarkers are the same or of the same type. Preferably, the differences can be represented as ratios (such as quotients) of the measurement values of respective biomarkers derived from the individuals to which the existing substance has been administered to the measurement values of biomarkers corresponding to the above biomarkers in the non-administered individuals. For example, the data includes quotients obtained by dividing the measurement values of biomarker A in organs A derived from individuals to which the existing substance has been administered by the measurement values of biomarker A in organs A derived from non-administered individuals.

When the biomarker is transcriptome, all RNAs that can be analyzed by RNA-Seq may be used. Alternatively, the RNAs may be analyzed for their expression, and divided into subsets (modules) of data indicating the dynamics of each RNA with which the organ name and the gene name are linked using, for example, WGCNA (https://labs.genetics.ucla.edu/horvath/Coexpression-Network/Rpackages/WGCNA/). For each module divided by means of WGCNA, a Pearson's correlation coefficient with 1-of-K representation may be calculated for each existing substance to select a module with the highest absolute value of the correlation coefficient for each existing substance, and the RNA in each organ included in the selected module may be used as a biomarker.

Further, when the biomarker in response to the administration of an existing substance is transcriptome, the variation in transcriptome in each organ of the animals to which the existing substance has been administered compared with that of the animals to which the existing substance has not been administered can be obtained using DESeq2 analysis. For example, the expression levels of RNAs in each organ collected from animals to which the existing substance has been administered and the expression levels of genes in each corresponding organ collected from animals to which the existing substance has not been administered are quantified by htseq-count to obtain respective count data. Then, respective organs and the expression levels of respective genes in respective organs are compared. As a result of the comparison, a $\log_2$ (fold) value of the variation in gene expression in the animals to which the existing substance has been administered and a p-value, which serves as an index of the probability of each variation, are output for each gene in each organ. Based on the $\log_2$ (fold) value, it is possible to determine whether or not the dynamics of a biomarker such as transcriptome is present.

The measurement values of a biomarker can be acquired by a known method. When the biomarker is a nucleic acid, the measurement values can be acquired by sequencing such as RNA-Seq, quantitative PCR, or the like. When the biomarker is a carbohydrate, lipid, glycolipid, amino acid, polyphenols; chemokine; at least one metabolic substance selected from the group consisting of ultimate metabolites, intermediary metabolites and synthetic raw material substances of the above-mentioned substances or the like, the measurement values can be acquired by, for example, mass spectrometry. When the biomarker is a glycoprotein, lipoprotein, peptide, protein or the like, the measurement values can be acquired by, for example, an ELISA (Enzyme-Linked Immuno Sorbent Assay) method. The method for collecting tissues, cells or body fluids derived from organs for use in the measurement and the preprocessing method for the measurement of a biomarker are also known.

The "test substance" is a substance to be evaluated for its actions. The test substance may be an existing substance, an equivalent of an existing substance or a new substance. In the prediction method, even when the relationship between an action of the test substance and an action of an existing substance or an equivalent substance of an existing substance has not been found, it is possible to predict an action of the test substance on humans. On the other hand, when the test substance is one selected from existing substances or equivalents of existing substances, unknown action of the existing substance or equivalent of the existing substance can be found. The unknown action may be one action or multiple actions. The unknown action is preferably a new indication. By predicting a new indication for a test substance in humans, drug repositioning can be also achieved. Administration of a test substance to non-human animals is known. Also, the data indicating the dynamics of a biomarker in one or multiple organs collected from non-human animals to which a test substance has been administered can be acquired in the same manner as the data indicating the dynamics of a biomarker in one or multiple organs collected from non-human animals to which an existing substance has been administered.

2. Construction of artificial intelligence model

2-1. Generation of training data

(1) Generation of first training data set

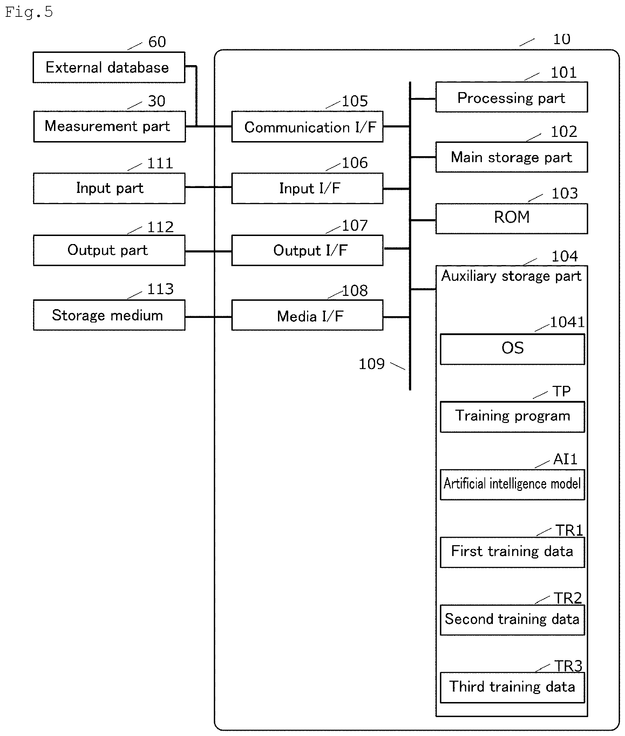

A first training data set is constituted of a set of data indicating the dynamics of a biomarker in one organ or each of multiple different organs, and the labels indicating the names of existing substances. The one organ or the multiple different organs may be collected from respective non-human animals to which multiple existing substances with a known action on humans have been individually administered. The first training data set may be stored in an auxiliary storage part 104 of a training device 10 shown as a database TR1 in FIG. 5.

The set of data indicating the dynamics of a biomarker in one organ or each of multiple different organs may be acquired by a method described in Section 1.(4) above.

Each item of the data indicating the dynamics of a biomarker in the respective organs may be linked with information about the names of existing substances administered, information about the names of organs collected, information about the names of biomarkers, and so on. The "information about the names" may be the names themselves or labels of abbreviated names or the like, or may be label values corresponding to respective names.

Each item of data included in the set of data indicating the dynamics of a biomarker serves as an element that constitutes a matrix in a first training data set for an artificial intelligence model, which is described later. When the biomarker is transcriptome, the expression level of each RNA corresponds to data included in the set of data indicating the dynamics of a biomarker, and serves as an element of a matrix constituting the first training data set. For example, when the biomarker is transcriptome, a $\log_2$ (fold) value of each existing substance obtained by DESeq2 analysis may be used as each element of the first training data set.

An example of the first training data set is as shown in Section 1.(1) above and FIG. 3(A).

As a set of data indicating the dynamics of a biomarker, measurement values of the biomarker may be directly used as elements of the first training data set, or may be subjected to standardization, dimensionality reduction or the like before being used as elements of the first training data set. An example of a standardization method is to transform data indicating expression differences such that the mean value is 0 and the variance is 1, for example. The mean value in the standardization can be the mean value in each organ, the mean value in each gene, or the mean value of all data. Also, the dimensionality reduction can be achieved by statistical processing such as a principal component analysis. The parent population in performing statistical processing can be set for each organ, for each gene, or for all data. For example, when the biomarker is transcriptome, only the genes having a p-value not greater than a predetermined value relative to a $\log_2$ (fold) value of each existing substance obtained by DESeq2 analysis may be used as the elements of the first training data set. The predetermined value can be $10^{-3}$ or $10^{-4}$, for example. Preferred is $10^{-4}$.

The labels indicating respective names of the predetermined existing substances administered included in the first training data set may be the names of the substances themselves or may be encoded.

The first training data set may be updated in response to the update of the existing substances or the addition of data indicating the dynamics of a new biomarker.

(2) Generation of second training data set

As shown in Section 1.(1) above and FIG. 3(B), the second training data set is generated by linking labels indicating respective names of multiple predetermined existing substances administered to non-human animals to generate the first training data set with labels indicating the indications reported for each of the multiple predetermined existing substances. For the indications for existing substances, by conducting, for each existing substance, a search for a word indicating the name of the existing substance, for example, labels of the names of indications corresponding thereto can be acquired from an external database such as FAERS, all drug labels of DAILYMED, Medical Subject Headings, Drugs@FDA, or International Classification of Diseases as described in Section 1.(4) above. There may be one indication or two or more indications per existing substance. When there are two or more indications per existing substance, the two or more multiple indications constitute the second training data set. The labels indicating the indications reported for each of the multiple predetermined existing substances can be acquired by performing text extraction, natural language processing, digitize processing, image analysis processing or the like on the data set stored in a database. For example, when labels indicating the names of respective indications corresponding to respective existing substances administered to non-human animals to generate the first training data set which are stored in an external database are registered as inserts in texts, syntactic analysis, word segmentation, semantic analysis or the like may be performed on the registered texts by natural language processing before the extraction of the texts corresponding to actions.

(3) Generation of third training data set

As described in Section 1.(1) above and FIG. 3(C), the third training data is a set of data in which the labels indicating indications reported for each of the multiple predetermined existing substances administered to acquire the first training data set as shown in FIG. 3(B) are linked with information about adverse events reported correspondingly to each of these indications. For the indications reported for each of the multiple predetermined existing substances, by conducting, for each existing substance, a search for a word of the name of the existing substance, for example, labels of the names of indications corresponding thereto can be acquired from an external database such as FAERS, all drug labels of DAILYMED, Medical Subject Headings, Drugs@FDA, or International Classification of Diseases. The labels indicating adverse events reported correspondingly to each of these indications can be acquired from an external database such as FAERS or clinicaltrials.gov by conducting a search for labels indicating the indication names. Also, when labels indicating the names of indications or adverse events are registered as inserts in texts, syntactic analysis, word segmentation, semantic analysis or the like may be performed on the registered texts by natural language processing before the extraction of the texts corresponding to actions.

The frequencies of occurrence of adverse events can be calculated by a method described in Section 1.(4) above.

(4) Generation of fourth training data set

As described in Section 1.(1) above and FIG. 3(D), the fourth training data set is generated by substituting the frequencies of occurrence of adverse events reported for indications corresponding to the labels indicating the names of existing substances administered to acquire the first training data (the frequencies of occurrence of adverse events in the second and subsequent columns shown in FIG. 3(C)) into the parts of the labels indicating the drug names included in the first training data set (the first column of FIG. 3(A) indicating the drug names).

2-2. Input of training data into artificial intelligence model

The artificial intelligence model is not limited as long as the problem associated with the present invention can be solved. In this embodiment, the use of an artificial intelligence model that can perform Link Prediction is preferred. Examples of such an artificial intelligence model include One-Class SVM (One-Class support vector machine).

An example of inputting fourth training data is described using a case where Link Prediction is performed with a One-Class SVM as an example. The data to be input into the One-class SVM are input into the One-class SVM as a fourth training data set obtained by associating the first training data set with the third training data set by a kernel function of the following equation:

$$k(g_A d_1, g_B d_2) = <g_A, g_B><d_1, d_2>$$

Here, $<\cdot, \cdot>$ denotes an operator that scales each vector such that 12 norms are equal to 1 and takes an inner product between both the scaled vectors.

As the One-class SVM, 'scikit-learn' package from Python, for example, may be used with a parameter nu=0.1.

2-3. System for training artificial intelligence model

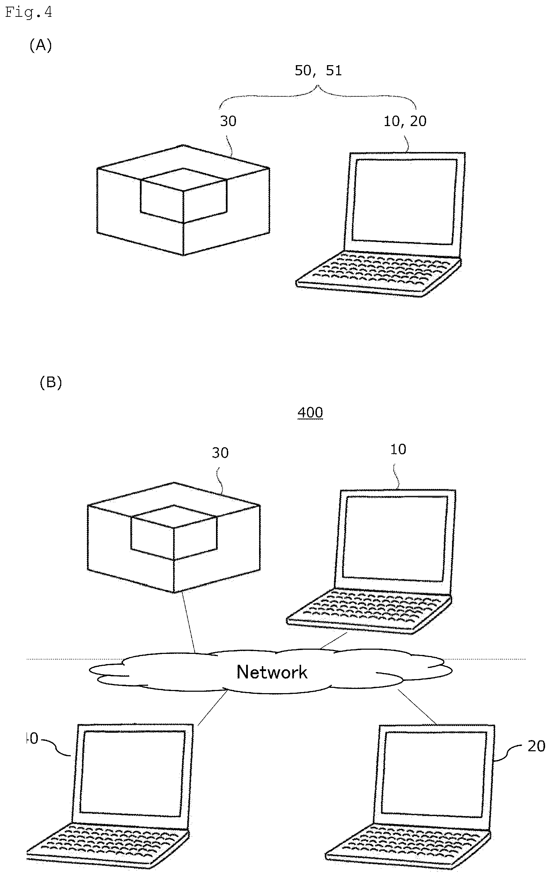

FIG. 4(A) illustrates a hardware configuration of a training system 50. The training system 50 includes a measurement part 30, which is a next-generation sequencer or the like, for acquiring measurement data of a biomarker, and a training device 10. The training device 10 may be communicably connected to the measurement part 30 by a wireless or wired network, or may acquire data acquired by the measurement part 30 via a storage medium such as a CD-R.

(1) Device for training artificial intelligence model

The training of the artificial intelligence model can be carried out using the training device 10 (which may be hereinafter referred to also as "device 10"), for example.

In the description of the device 10 and the processing in the device 10, for the terms that are common to those described in Sections 1. and 2-1. above, the above description is incorporated here.

The device 10 includes at least a processing part 101 and a storage part. The storage part is constituted of a main storage part 102 and/or an auxiliary storage part 104.

FIG. 5 illustrates a hardware configuration of the device 10. The device 10 may be connected to an input part 111, and an output part 112 and a storage medium 113. The device 10 may be also connected to a measurement part 30, which is a next-generation sequencer, a mass spectrometer or the like. Also, the device 10 may be communicably connected to an external database 60 such as FAERS, all drug labels of DAILYMED, Medical Subject Headings, Drugs@FDA, International Classification of Diseases or clinicaltrials.gov.

In the device 10, the processing part 101, the main storage part 102, a ROM (read only memory) 103, the auxiliary storage part 104, a communication interface (I/F) 105, an input interface (I/F) 106, an output interface (I/F) 107 and a media interface (I/F) 108 are connected for mutual data communication by a bus 109.

The processing part 101 is constituted of a CPU, MPU or the like. The processing in the processing part 101 may be assisted by a GPU. The processing part 101 executes a computer program stored in the auxiliary storage part 104 or the ROM 103, and processes the acquired data, whereby the device 10 functions. The processing part 101 acquires a data set indicating the dynamics of a biomarker in multiple different organs collected from non-human animals to which an existing substance has been administered as described in Section 1. above and known actions of the existing substance on humans as training data. Also, the processing part 101 trains an artificial intelligence model using the two types of training data.

The ROM 103 is constituted of a mask ROM, a PROM, an EPROM, an EEPROM or the like, and stores computer programs that are executed by the processing part 101 and data that are used thereby. The ROM 103 stores a boot program that is executed by the processing part 101 when the device 10 is started up, programs and settings relating to the operation of the hardware of the device 10, and so on.

The main storage part 102 is constituted of a RAM (Random access memory) such as an SRAM or DRAM. The main storage part 102 is used to read out the computer programs stored in the ROM 103 and the auxiliary storage part 104. The main storage part 102 is also utilized as a workspace when the processing part 101 executes these computer programs. The main storage part 102 temporarily stores training data or the like acquired via a network, functions of the artificial intelligence model read out by the auxiliary storage part 104, and so on.

The auxiliary storage part 104 is constituted of a hard disk, a semiconductor memory element such as a flash memory, an optical disc, or the like. In the auxiliary storage part 104, various computer programs to be executed by the processing part 101 and various setting data for use in executing the computer programs are stored. Specifically, the auxiliary storage part 104 stores operation software (OS) 1041, a training program TP, an artificial intelligence model database AI1, a database TR1 for storing a first training data set, a database TR2 for storing a second training data set, a database TR3 for storing a third training data set in a non-volatile manner. The training program TP performs processing for training an artificial intelligence as described later in cooperation with the operation software (OS) 1041.

The communication I/F 105 is constituted of a serial interface such as a USB, IEEE1394 or RS-232C, a parallel interface such as a SCSI, IDE or IEEE1284, and an analog interface constituted of a D/A converter, A/D converter or the like, a network interface controller (NIC) and so on. The communication I/F 105 functions as a communication part 105, and, under the control of the processing part 101, receives data from the measurement part 30 or other external devices, and, when necessary, transmits information stored in or generated by the device 10 to the measurement part 30 or to the outside, or displays it. The communication I/F 105 may communicate with the measurement part 30 or other external devices (not shown, e.g., other computers or cloud systems) via a network.

The input I/F 106 is constituted of a serial interface such as a USB, IEEE1394 or RS-232C, a parallel interface such as an SCSI, IDE or IEEE1284, an analog interface constituted of a D/A converter, A/D converter or the like, and so on. The input I/F 106 accepts character input, clicks, sound input or the like from the input part 111. The accepted inputs are stored in the main storage part 102 or the auxiliary storage part 104.

The input part 111 is constituted of a touch panel, keyboard, mouse, pen tablet, microphone or the like, and performs character input or sound input into the device 10. The input part 111 may be externally connected to the device 10 or may be integrated with the device 10.

The output I/F 107 is constituted, for example, of an interface similar to that for the input I/F 106. The output I/F 107 outputs information generated by the processing part 101 to the output part 112. The output I/F 107 outputs information generated by the processing part 101 and stored in the auxiliary storage part 104 to the output part 112.

The output part 112 is constituted, for example, of a display, a printer or the like, and displays measurement results transmitted from the measurement part 30, various operation windows in the device 10, respective items of training data, an artificial intelligence model, and so on.

The media I/F 108 reads out, for example, application software or the like stored in the storage medium 113. The read out application software or the like is stored in the main storage part 102 or the auxiliary storage part 104. Also, the media I/F 108 writes information generated by the processing part 101 into the storage medium 113. The media I/F 108 writes information generated by the processing part 101 and stored in the auxiliary storage part 104 into the storage medium 113.

The storage medium 113 is constituted of a flexible disk, a CD-ROM, a DVD-ROM or the like. The storage medium 113 is connected to the media I/F 108 by a flexible disk drive, a CD-ROM drive, a DVD-ROM drive or the like. An application program or the like for a computer to execute an operation may be stored in the storage medium 113.

The processing part 101 may acquire application software and various settings necessary for control of the device 10 via a network instead of reading them out of the ROM 103 or the auxiliary storage part 104. It is also possible that the application program is stored in an auxiliary storage part of a server computer on a network and the device 10 accesses this server computer to download the computer program and stores it in the ROM 103 or the auxiliary storage part 104.

Also, in the ROM 103 or the auxiliary storage part 104, an operation system that provides a graphical user interface environment, such as Windows (trademark) manufactured and sold by Microsoft Corporation in the United States, has been installed, for example. An application program according to a second embodiment shall operate on the operating system. In other words, the device 10 may be a personal computer or the like.

(2) Processing for training artificial intelligence model

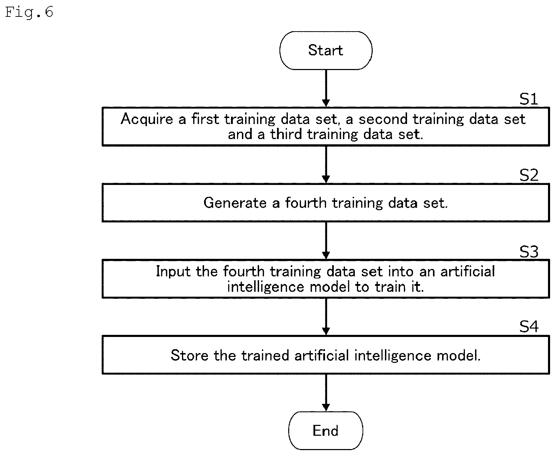

Referring to FIG. 6, the flow of processing for training an artificial intelligence model by the training program TP is described.

The processing part 101 accepts a command to start processing input by an operator through the input part 111, and, in step S1, acquires the first training data set, the second training data set and the third training data set from the first training data set database TR1, the second training data set database TR2, the third training data set database TR3, respectively, stored in the auxiliary storage part 104.

Next, the processing part 101 accepts a command to start generation of a fourth training data set input by the operator through the input part 111, and, in step S2, generate a fourth training data set.

Next, the processing part 101 accepts a command to input the fourth training data set input by the operator through the input part 111, and, in step S3, inputs the fourth training data set into the artificial intelligence model AI1 to train the artificial intelligence model.

The processing part 101 stores the trained artificial intelligence model in the auxiliary storage part 104.

The transition from one step to another may be made according to a command input by the operator, or may be triggered by the completion of the previous step so that the processing part 101 can make the transition automatically.

In the training processing, for the terms and explanations that are common to those described in Sections 1. and 2-1. above, the above description is incorporated here.

3. Prediction of indication by artificial intelligence model

3-1. Generation of test data

(1) Generation of first test data set

A first test data set is a set of data indicating the dynamics of a biomarker in one organ or each of multiple different organs, and may be acquired from one or multiple organs corresponding to one organ or multiple different organs from which the first training data have been acquired. The set of data indicating the dynamics of a biomarker in respective organs may be acquired in the same manner as a data set indicating the dynamics of a biomarker that is used as first training data by a method as described in Section 1.(4) above.

(2) Generation of second test data set

As described in Section 1.(2) above, the second test data is a set of data in which labels of multiple known indications are linked with information about adverse events reported correspondingly to each of the multiple known indications. The labels of multiple known indications and labels indicating adverse events reported correspondingly to each of these indications can be acquired from an external database such as FAERS or clinicaltrials.gov by conducting a search for labels indicating the indication names. Also, when the labels indicating the names of indications or adverse events are registered as inserts in texts, syntactic analysis, word segmentation, semantic analysis or the like may be performed on the registered texts by natural language processing before the extraction of the texts corresponding to actions.

The frequencies of occurrence of adverse events can be calculated by a method described in Section 1.(4) above.

3-2. Prediction system 1

FIG. 4(A) illustrates a hardware configuration of a prediction system 51. The prediction system 51 includes a measurement part 30, which is a next-generation sequencer or the like, for acquiring measurement data of a biomarker, and a prediction device 20. The prediction device 20 may be connected to the measurement part 30 via a wireless or wired network, or may acquire data acquired by the measurement part 30 via a storage medium such as a CD-R.

(1) Indication prediction device

The prediction of indications can be achieved using the prediction device 20 (which may be hereinafter referred to simply as "device 20"), for example,

In the description of the device 20 and the processing in the device 20, for the terms that are common to those described in Sections 1. and 2-1. above, the above description is incorporated here.

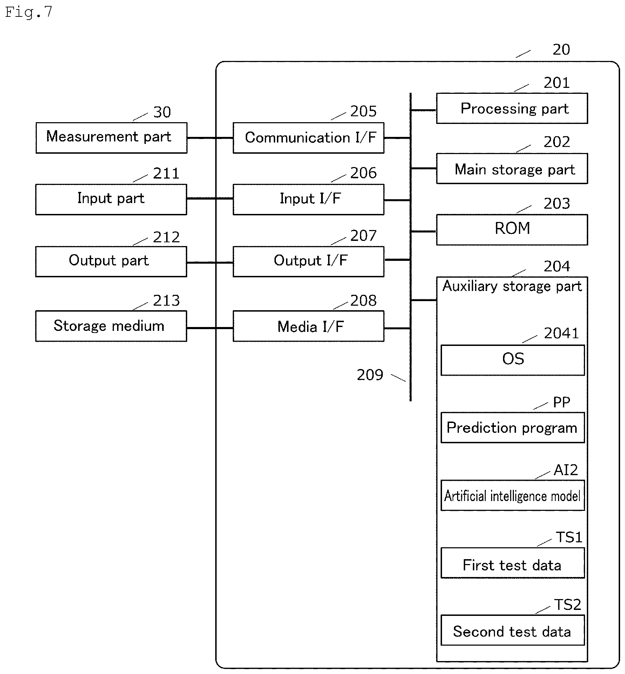

FIG. 7 illustrates a hardware configuration of the prediction device 20 (which may be hereinafter referred to also as "device 20"). The device 20 includes at least a processing part 201 and a storage part. The storage part is constituted of a main storage part 202 and/or an auxiliary storage part 204. The device 20 may be connected to an input part 211, an output part 212, and a storage medium 213. Also, the device 20 may be connected to the measurement part 30, which is a next-generation sequencer, a mass spectrometer or the like.

In the device 20, the processing part 201, the main storage part 202, a ROM (read only memory) 203, the auxiliary storage part 204, a communication interface (I/F) 205, an input interface (I/F) 206, an output interface (I/F) 207, and a media interface (I/F) 208 are connected for mutual data communication by a bus 209.

Because the basic hardware configuration of the device 20 is the same as that of the device 10, the description in Section 2-3.(1) above is incorporated here. The communication interface 205 functions as a communication part 205.

However, in the auxiliary storage part 204 of the device 20, operation software (OS) 2041, a prediction program PP, a trained artificial intelligence model AI2, a database TS1 for storing a first test data set, and a database TS2 for storing a second test data set are stored in a non-volatile manner in place of the operation software (OS) 1041, the training program TP, the artificial intelligence model AI1, the database TR1 for storing a first training data set, the database TR2 for storing a second training data set and the database TR3 for storing a third training data set. The prediction program PP performs indication prediction processing as described later in cooperation with the operation software (OS) 2041.

(2) Processing for predicting indication

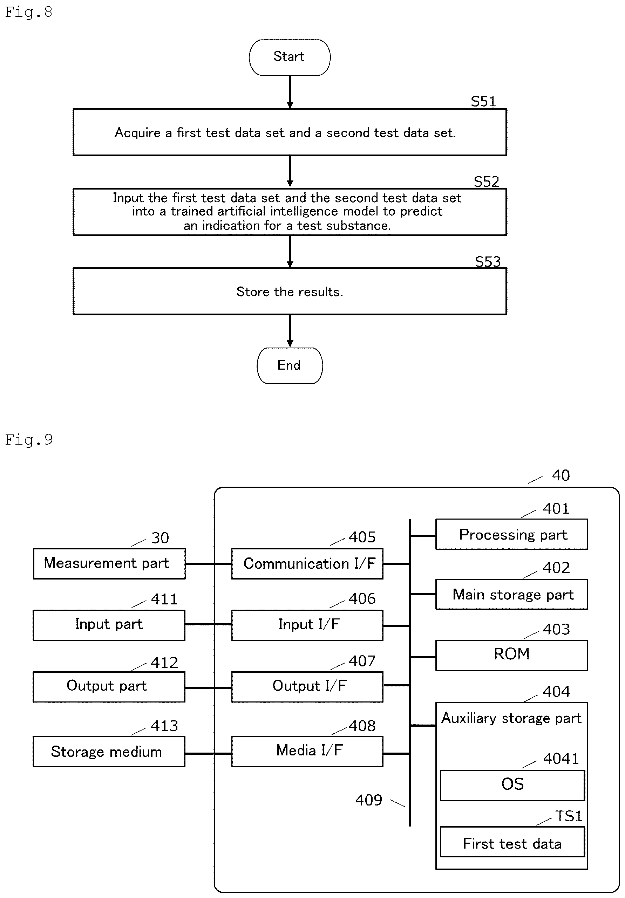

Referring to FIG. 8, the flow of processing for predicting an indication by the prediction program PP is described.

The processing part 201 accepts a command to start processing input by an operator through the input part 211, and, in step S51, acquires the first test data set and the second test data set stored in the auxiliary storage part 204.

Next, the processing part 201 accepts a command to start prediction input by the operator through the input part 211, and, in step S52, inputs the first test data set database TS1, the second test data set database TS2, the first test data set and the second test data set into the trained artificial intelligence model AI2 to predict an indication for the test substance.

At this time, the trained artificial intelligence model AI2 determines one by one whether or not the test substance of interest is effective against all the indications input as the second test data individually. Specifically, the trained artificial intelligence model AI2 determines whether or not there is a link between the drug of interest and the individual indications in an LP problem.

Next, the processing part 201 stores the results in the storage part. A result that the processing part 201 derives from the trained artificial intelligence model AI2 is a label "1" if the test substance is effective against a certain indication and a label "-1" if the test substance is not effective against a certain indication.

In other words, the indications marked with "1" are indications predicted for the test substance.

Further, when the artificial intelligence model is a One-Class SVM, decision function values, which indicate the reliability of the prediction, are calculated. When many indications are output as prediction results, it is possible to predict that a higher value indicates a more likely indication. Also, when many indications are output as prediction results, prediction may be made in the same manner using data indicating the dynamics of transcriptome in one or multiple organs collected after the administration of a drug with an action mechanism similar to that of the test substance of interest as a test substance. Then, the indications found to be common to the prediction result for the test substance of interest and the prediction result for the other test substance with a similar action mechanism by comparison therebetween may be used as prediction results.

3-3. Prediction system 2

FIG. 4(B) shows the configuration of a prediction system 400.

The prediction system 400 is communicably connected to a measurement part 30, the training device 10, the prediction device 20, and a server device 40 that transmits a data set indicating the dynamics of a biomarker. The training device 10 and the prediction device 20 acquire data acquired by the measurement part 30 via the server device 40.

(1) Server device

Regarding the server device 40 (which may be hereinafter referred to simply as "device 40"), for the terms that are common to those described in Sections 1. and 2-1. above, the above description is incorporated here.

FIG. 9 shows a hardware configuration of the server device 40 (which may be referred to also as "device 40"). The device 40 includes at least a processing part 401 and a storage part. The storage part is constituted of a main storage part 402 and/or an auxiliary storage part 404. The device 40 may be connected to an input part 411, an output part 412 and a storage medium 413. Also, the device 40 may be communicably connected to a measurement part 30, which is a next-generation sequencer, a mass spectrometer or the like, by a wired or wireless network.

In the device 40, the processing part 401, the main storage part 402, a ROM (read only memory) 403, the auxiliary storage part 404, a communication interface (I/F) 405, an input interface (I/F) 406, an output interface (I/F) 407 and a media interface (I/F) 408 are connected for mutual data communication by a bus 409.

Because the basic hardware configuration of the device 40 is the same as that of the device 10, the description in Section 2-3.(1) above is incorporated here. The communication interface 405 functions as a communication part 405.

However, in the auxiliary storage part 404 of the device 40, operation software (OS) 4041, a database TS1 for storing a first test data set are stored in a non-volatile manner in place of the operation software (OS) 1041, the training program TP, the artificial intelligence model AI1, the database TR1 for storing a first training data set, the database TR2 for storing a second training data set and the database TR3 for storing a third training data set.

(2) Operation of prediction system 2

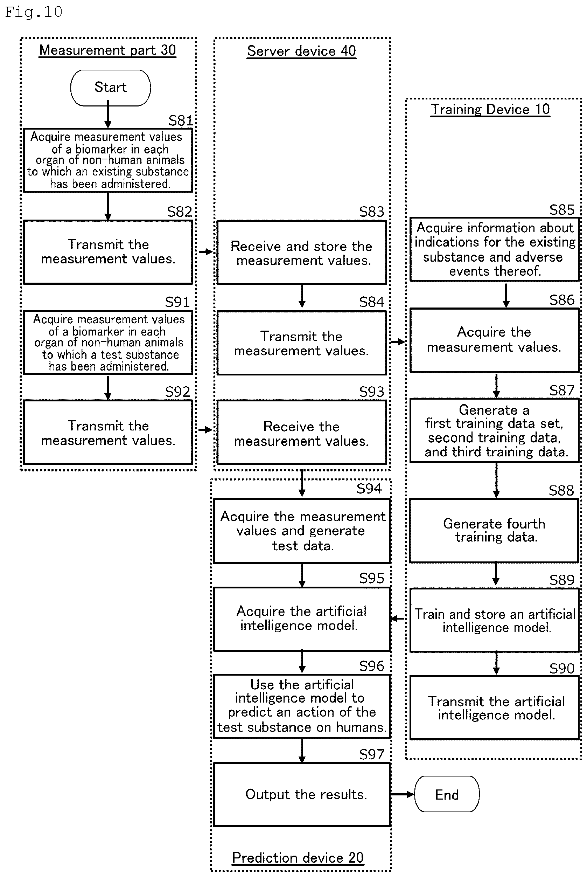

Referring to FIG. 10, the operation of the prediction system is described.

Here, the sequential flow from the acquisition of measurement values of a biomarker by the measurement part 30 to the output of prediction results is described.

In step S81, the measurement part 30 acquires measurement values of a biomarker in each organ of non-human animals to which an existing substance has been administered. The acquisition of measurement values in the measurement part 30 may be made in response to the input of a command to start measurement by an operator. In step S82, the measurement part 30 transmits the acquired measurement values to the server device 40. The transmission processing may be performed in response to the input of a command to start transmission by the operator.

In step S83, the processing part 401 of the server device 40 acquires the measurement values via the communication I/F 405. At this time, the communication I/F 405 functions as a communication part.

In step S84, in response to a command to start acquisition of measurement values input by the operator through the input part 111 of the training device 10, the processing part 101 of the training device 10 transmits a signal for starting transmission of measurement values from the communication I/F 105 to the server device 40. The processing part 401 of the server device 40 accepts the input for starting transmission of measurement values via the communication I/F 405, and starts transmission of the measurement values from the communication I/F 405. At this time, the communication I/F 105 and the communication I/F 405 function as a communication part 105 and a communication part 405, respectively.

In step S85, the processing part 101 of the training device 10 acquires information about indications for the existing substance administered to non-human animals and adverse events corresponding to the indications from the external database 60 via the communication I/F 105.

Also, in step S84, the processing part 101 of the training device 10 acquires the measurement values transmitted from the server device 40 via the communication I/F 105 (step S86), and stores the measurement values in the storage part of the training device 10. Step S86 may be performed prior to step S85.

Next, in step S87 of FIG. 10, the processing part 101 of the training device 10 generates a first training data set, a second training data set and a third training data set in accordance with the processing shown in step S1 of FIG. 6. The description of step S1 in FIG. 6 is incorporated here.

Next, in step S88 of FIG. 10, the processing part 101 of the training device 10 generates a fourth training data set from the first training data set, the second training data set and the third training data set in accordance with the processing shown in step S2 of FIG. 6. The description of step S2 in FIG. 6 is incorporated here.

Next, in step S89 of FIG. 10, the processing part 101 of the training device 10 inputs the fourth training data set into an artificial intelligence model in accordance with the processing shown in steps S3 to S4 of FIG. 6 to train the artificial intelligence model, and stores the trained artificial intelligence model in the storage part. The description of steps S3 to S4 of FIG. 6 is incorporated here.

After accepting a command to start transmission of the artificial intelligence model from the prediction device 20, the processing part 101 of the training device 10 transmits the trained and stored artificial intelligence model to the prediction device 20 via the communication I/F 105 in step S90 of FIG. 10. At this time, the communication I/F 105 functions as a communication part 105.

Next, in step S91, the measurement part 30 acquires measurement values of a biomarker in each organ of non-human animals to which the test substance has been administered. The acquisition of measurement values in the measurement part 30 may be made in response to the input of a command to start measurement by the operator. In step S92, the measurement part 30 transmits the acquired measurement values to the server device 40. The transmission processing may be performed in response to the input of a command to start transmission by the operator.

In step S93, the processing part 401 of the server device 40 acquires the measurement values via the communication I/F 405. At this time, the communication I/F 405 functions as a communication part 405.

In step S94, in response to a command to start acquisition of measurement values input by the operator through the input part 211 of the prediction device 20, the processing part 201 of the prediction device 20 transmits a signal for starting transmission of measurement values from the communication I/F 205 to the server device 40. The processing part 401 of the server device 40 accepts the input for starting transmission of measurement values via the communication I/F 405, and starts transmission of the measurement values from the communication I/F 405. At this time, the communication I/F 205 and the communication I/F 405 function as a communication part. The processing part 201 of the prediction device 20 acquires the measurement values via the communication I/F 205, and stores the measurement values in the storage part of the prediction device 20. Subsequently, the processing part 201 of the prediction device 20 generates a first test data set. The first test data set is generated as described in Section 2-4.(1) above.

Next, in step S95, the processing part 201 of the prediction device 20 transmits a command to start transmission of an artificial intelligence model to the training device 10 via the communication I/F 205. When accepting the command to start transmission of an artificial intelligence model from the prediction device 20, the processing part 101 of the training device 10 transmits the trained artificial intelligence model to the prediction device 20 via the communication I/F 105 of the training device 10. The prediction device 20 acquires the trained artificial intelligence model via the communication I/F 205. Step S95 may be performed prior to step S94.

Next, in step S96, the processing part 201 of the prediction device 20 inputs the first test data generated in step S94 and the second test data stored in the storage part into the trained artificial intelligence model AI2 acquired in step S95, and predicts actions of the test substance on humans in accordance with step S52 of FIG. 8. In step S97, the processing part 201 of the prediction device 20 outputs the results. Alternatively, in steps S94 to 97 of FIG. 10 the processing part 201 of the prediction device 20 may predict a prediction result relating to new indications for an existing substance.

(3) Method for constructing prediction system

A method for constructing a prediction system includes the step of preparing the training device 10 and the prediction device 20. The constructing method may further include the step of preparing measurement values of a biomarker in one or multiple organs of non-human animals to which an existing substance has been administered, or measurement values of a biomarker in one or multiple organs of non-human animals to which a test substance has been administered.

## 4. Computer program

### 4-1. Training program

The training program TP is a computer program that causes a computer to execute the processing including steps S1 to S4 of FIG. 6 described above in connection with the training of an artificial intelligence model to cause the computer to function as the training device 10.

### 4-2. Prediction program

The prediction program PP is a computer program that causes a computer to execute the processing including steps S51 to S53 described above in connection with prediction of actions of a test substance to cause the computer to function as the prediction device 20.

## 5. Storage medium having computer programs stored therein

This section relates to a storage medium having the above computer programs stored therein. The computer programs are stored in a storage medium such as a hard disk, a semiconductor memory element such as a flash memory, or an optical disc. Also, the computer programs may be stored in a storage medium connectable via a network such as a cloud server. The computer programs may be program products in a downloadable form or stored in a storage medium.

The storage format of the programs in the storage medium is not limited as long as a device as described above can read the programs. The storage in the storage medium is preferably in a non-volatile manner.

## 6. Modifications

In Section 2. above, an embodiment is shown in which the training device 10 and the prediction device 20 are different computers. However, one computer may perform training of an artificial intelligence model and prediction.

In this specification, the same reference numeral attached to hardware indicates the same part or same function.

## Examples

Examples are shown below to describe the present invention in more detail. However, the present invention should not be construed as being limited to the following embodiments.

The following animal experiments were conducted on approval of the Ethics Committee of Karydo TherapeutiX, Inc.

## Experimental example I. Gene expression analysis in drug-administered mice

### 1-1. Preparation of drug-administered mice, and gene expression analysis

#### 1. Administration of drugs

##### (1) Alendronate

A solution of alendronate sodium salt trihydrate (Wako) in PBS (NACALAI TESQUE, INC.) was subcutaneously injected to 11-week old male C57BL/6N mice in a dose of 1.0 mg/kg every 3 or 4 days for 8 days. The drug was newly prepared for each administration. Each organ was collected in the afternoon of the eighth day after the drug administration.

##### (2) Acetaminophen

10-week old male C57BL/6N mice were fasted for 12 hours, during which they were allowed to take water freely. Immediately after the fasting period, acetaminophen (Wako) dissolved in saline (Otsuka Pharmaceutical Co., Ltd.) was intraperitoneally administered to the mice in a single dose of 300 mg/kg. After the administration, the mice were allowed to take normal diet freely. The administration was done by noon, and organs were collected two hours after the administration.

##### (3) Aripiprazole

A solution of aripiprazole (Sigma-Aldrich) in a 0.5% (w/v) carboxymethyl cellulose 400 solution (Wako) was intraperitoneally administered to 11-week old male C57BL/6N mice in a single dose of 0.3 mg/kg. The drug was administered in the afternoon, and organs were collected two hours later.

##### (4) Asenapine

A solution of asenapine maleate (Chemscene) in saline was subcutaneously administered to 11-week old male C57BL/6N mice in a single dose of 0.3 mg/kg. The drug was administered in the afternoon, and organs were collected two hours later.

##### (5) Cisplatin

Cisplatin (Bristol-Myers Squibb) was intraperitoneally administered in a single dose of 20 mg/kg to 11-week old male C57BL/6N mice. Organs were collected in the afternoon of the third day after the drug administration.

##### (6) Clozapine

Clozapine (Sigma-Aldrich) was subcutaneously administered in a single dose of 0.3 mg/kg to 11-week old male C57BL/6N mice. The clozapine was first dissolved in acetic acid, and then diluted with saline and adjusted to pH 6 with 1M NaOH. Organs were collected in the afternoon two hours after the drug administration.

(7) Doxycycline

9-Week old male C57BL/6N mice were fed with RO water containing 5% sucrose (NACALAI TESQUE, INC.) and 2 mg/mL of doxycycline hydrochloride n-hydrate (Wako) for 2 weeks. The RO water containing the drug was replaced with new one every week. Organs were collected in the afternoon of the 13th day after the drug administration. The negative control group was fed with RO water containing 5% of sucrose (NACALAI TESQUE, INC.).

(8) Empagliflozin

Empagliflozin (Toronto research chemical) dissolved in 0.5% carboxymethyl cellulose was forcibly administered orally to 10-week old male C57BL/6N mice in a daily dose of 10 mg/kg for 2 weeks. The drug was newly prepared for each administration. Organs were collected in the afternoon of the 14th day after the start of the drug administration.

(9) Lenalidomide

Lenalidomide (Wako) was dissolved in a solution containing 0.5% of carboxymethyl cellulose and 0.25% of Tween-80 (NACALAI TESQUE, INC.), and the solution was forcibly administered orally to 8-week old male C57BL/6N mice in a daily dose of 50 mg/kg for 69 days. The drug was newly prepared for each administration. Organs were collected in the afternoon of the 69th day after the start of the drug administration. A solution containing 0.5% of carboxymethyl cellulose and 0.25% of Tween-80 was administered to the negative control group.

(10) Lurasidone

Lurasidone hydrochloride (Medchemexpress) dissolved in a 0.5% carboxymethyl cellulose solution was forcibly administered orally to 11-week old male C57BL/6N mice in a single dose of 0.3 mg/kg. Organs were collected in the afternoon two hours after the drug administration.

(11) Olanzapine

Olanzapine (Tokyo Chemical Industry Co., Ltd.) dissolved in a 0.5% carboxymethyl cellulose solution was forcibly administered orally in a single dose of 0.3 mg/kg. Organs were collected in the afternoon two hours after the drug administration.

(12) Evolocumab (Repatha (trademark))

Repatha (trademark) (Astellas Pharma Inc.) dissolved in saline was subcutaneously administered to 11-week old male C57BL/6N mice in a dose of 10 mg/kg every 10 days for 4 weeks. Organs were collected in the afternoon 4 weeks after the drug administration.

(13) Ricedronate

Sodium risedronate salt (Cayman Chemical Company) dissolved in PBS was forcibly administered orally to 11-week old male C57BL/6N mice in a dose of 10 mg/kg every other day for 8 days. The drug was newly prepared for each administration. Organs were collected in the afternoon of the 8th day after the start of administration.

(14) Sofosbuvir

Sofosbuvir (LKT) was intraperitoneally administered to 7-week old male C57BL/6N mice in a daily dose of 20 mg/kg for 10 days. The sofosbuvir was first diluted with DMSO (NACALAI TESQUE, INC.), and then diluted with PBS at 100-folds before administration (the final concentration was 1.0% DMSO/PBS). Organs were collected in the afternoon of the 10th day after the start of administration.

(15) Teriparatide

Human parathyroid hormone fragment 1-34 (teriparatide) (Sigma-Aldrich) dissolved in saline was subcutaneously administered to 10-week old male C57BL/6N mice in a daily dose of 40 $\mu$g/kg. Organs were collected in the afternoon of the 14th day after the start of drug administration. Saline was administered to the negative controls.

(16) Wild type (WT) mice

The organs were collected in the afternoon from 11-week old male C57BL/6N mice to which no drug had been administered.

2. Mice and 24-organ transcriptome analysis

(1) Organs

The experiments using mice, the extraction of organs, and the transcriptome analysis were performed in accordance with the methods described in Patent Document 1. The 24 organs are adrenal gland, aorta, bone marrow cell (BM), brain, colon, eye, heart, ileum, jejunum, left kidney, liver, lung, pancreas, parotid gland, pituitary gland, skeletal muscles, skin, skull, spleen, stomach, left testicle, thymus, thyroid gland, and sexual gland white adipose tissue (WAT).

All mice were raised in a temperature-controlled room at approximately 25°C under alternating 12-hour light and dark cycles and allowed to freely take water and normal feed (CE-2, CLEA Japan, Inc., Tokyo, Japan).

(2) Transcriptome analysis

The transcriptome analysis was conducted using QuantSeq 3'mRNA-Seq Library Prep Kit for Illumina (FWD) (cat#015.384, LEXOGEN) and Illumina NextSeq 500 (75bp single-read, ca. 400 million reads/run, NextSeq 500/550 High Output Kit v2.5, cat#20024906).

Differential gene expression data of each organ collected from the mice to which each drug had been administered were used as characteristics of each drug for machine learning frame work. RNA-seq data processing (mapping and count of transcription products) was performed in accordance with the method described in Patent Document 1.

Mapping of mouse genome was performed on mm10 using TopHat2. Differential gene expression in each organ of the drug administered groups and the negative control groups (doxycycline and lenalidomide administered groups) or the WT mouse group (control against groups to which drugs other than doxycycline and lenalidomide had been administered) was identified by DESeq2(1.22.1). Each of the drug administered groups, the negative control groups and the WT mouse group was respectively analyzed with n=2.

3. Examples

An artificial intelligence model was constructed with Link Prediction (LP) using a One-class SVM to predict indications for the drugs.

3-1. Training

(1) First training data

As a characteristic of each drug, genes that showed a change in expression P<0.0001 in each organ were selected. Labels of organs, combinations of the $\log_2$fold values of all genes selected from all organs (24-organ frameworks) or individual organs (individual organ frameworks) and the organ names, and the name of the drug administered to acquire the gene expression data were made into a set and used as first training data.

(2) Second training data

The labels of the names of drugs administered to mice in Section 1. above and the labels of the indications for each of the drugs were made into a set and used as second training data. The names of indications corresponding to the drug names were in accordance with the FDA Adverse Event Reporting System (FAERS: https://open.fda.gov/data/faers/).

(3) Third training data

Adverse event report data from 2014 Q2 to 2018 Q1 were downloaded from FAERS (https://www.fda.gov/Drugs/GuidanceComplianceRegulatoryInformation/Surveillance/Adverse DrugEffects/ucm082193.htm). The words indicating adverse events corresponding to the names of indications for each drug administered to mice in Section 1. above were extracted from the report data. One word extracted was regarded as one reported adverse event, and the frequency of occurrence (%) of each adverse event was respectively calculated by means of the formula: (the number of cases in which one adverse event was reported for the name of an indication for one drug)/(the number of all adverse events reported for the name of an indication for one drug).

(4) Fourth training data

When the drug names are defined as A and B, for example, $g_A$ and $g_B$ respectively indicate the pattern of transcriptome observed in 24 organs when drugs A and B are administered (first training data set). Also, when an indication for drug A and an indication for drug B are represented by "1" and "2," respectively, and elements of adverse events (AEs) reported for the indication 1 are represented by i, ii ... N, the vectors of the indication 1 are represented as $d_1 = (d_{1i}, d_{1ii}, ... , d_{1N})$ and $d_2 = (d_{2i}, d_{2ii}, ... , d_{2N})$ (third training data set). Also, because the second training data set includes a set of a label indicating the name of drug A and a label indicating the name of indication 1 and a set of a label indicating the name of drug B and a label indicating the name of indication 2, the sets can be represented as $g_A d_1$ and $g_B d_2$, respectively (second training data set). Here, an indication is regarded as positive (indicated) when the number of records for drug A taken by patients with indication 1 exceeds 10 in FAERS.

(5) One-class SVM

As the data to be input into a One-class SVM, a fourth training data set obtained by associating the first training data set with the third training data set by a kernel function below was input into a One-class SVM

$$k(g_A d_1, g_B d_2) = <g_A, g_B><d_1, d_2>$$

Here, $<\cdot,\cdot>$ denotes an operator that scales each vector such that 12 norms are equal to 1 and takes an inner product between both the scaled vectors.

As the One-class SVM, p 'scikit-learn' package from Python was used with a parameter nu=0.1.

3-2. Prediction

The patterns of transcriptome in 24 organs in response to the administration of a drug of interest (first test data), and [labels indicating the names of all indications] and [combinations of the names of adverse events corresponding to the indications and the frequencies of occurrence thereof (gd)] registered in FAERS were input in the trained One-class SVM to cause the trained One-class SVM to determine whether or not the drug of interest would effective against all the indications separately. Specifically, in LP problem, the trained One-class SVM was caused to determine whether or not there was a link between the drug of interest and individual indications. The SVM returns a label "1" when the drug of interest is effective against a certain indication, and returns a label "-1" when the drug of interest is not effective against a certain indication.

3-3. Example 1

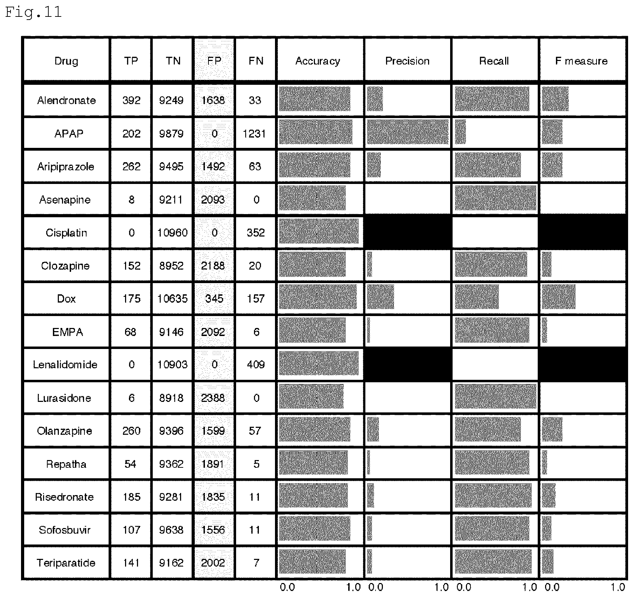

In Example 1, prediction was made assuming that indications for one of the drugs administered in Section 1. above were unknown. In other words, the One-class SVM was first trained using data on 14 drugs excluding one of the drugs administered in Section 1. above as training data. After that, the excluded drug was used as a drug of interest, and the patterns of transcriptome in response to the administration of the drug of interest as first test data were input in the trained One-class SVM together with the second test data to predict an indication. The results are shown in FIG. 11. In FIG. 11, TN represents true negative, TP represents true positive, FN represents false negative, and FP represents true positive. True negative indicates the number of items that were able to be predicted as not being indications for those that are not indications, and true positive indicates the number of items that were able to be predicted as being indications for those that are indications. False negative indicates the number of items that were predicted as being not indications for those that are indications, and false positive indicates the number of items that were predicted as being indications for those that are not indications. The accuracy score is a score that indicates the accuracy of prediction. The recall score is the coverage rate in the case of being predicted as "being an indication." The precision score indicates the reliability in the case of being predicted as "being an indication."

The accuracy score was high for all the 15 drugs (>0.78). These results indicate that 78% or more of predicted indications or non-indications have been actually reported or not reported. Also, the recall score showed a high value (>0.8) for alendronate, aripiprazole, asenapine, clozapine, empagliflozin, lurasidone, olanzapine, evolocumab, ricedronate, sofosbuvir and teriparatide. The recall scores indicate that 80% or more indications already reported for these drugs can be predicted. The recall score of doxycycline is 0.527, which indicates that about 50% of indications reported are predicted for this drug. Only acetaminophen (APAP) showed a high precision score (1.000), and others all showed a low precision score (<0.35). The precision score and the F major score were not able to be calculated for cisplatin and lenalidomide because both of them showed 0 TP and 0 FN. The reason for such a low precision score for many drugs was thought to be mainly due to the presence of more FPs compared to TPs.

These results indicate that the prediction method of the present invention is useful for predicting an indication for a new substance with no known indication.

3-4. Example 2

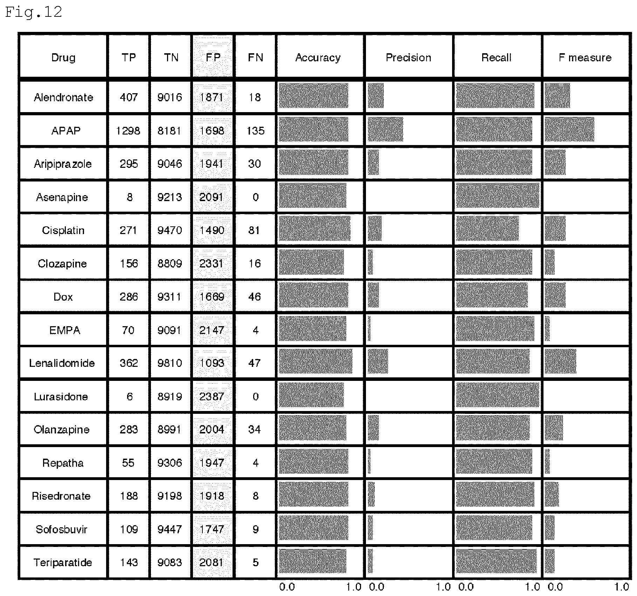

It was evaluated whether or not the present invention is useful for exploration of new indications for a known substance, i.e., what is called drug repositioning. An artificial intelligence was trained using data on all 15 drugs listed in Section 1. above to predict an indication for individual drugs. The results are shown in FIG. 12. The symbols in the figure are the same as those in FIG. 11.

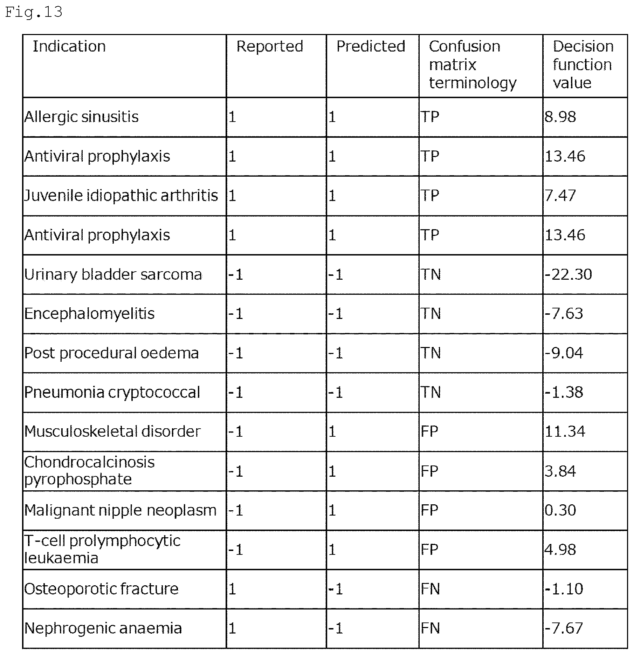

As a result, the number of TPs increased and the number of FNs decreased for all drugs. The recall scores also improved. Further, the accuracy score and the recall score improved for all drugs, with the range being between 0.770-1.000. These results indicate that both reported indications and unreported indications can be captured with a probability of 77% or more. The precision score was low for all drugs due to a large number of FNs. In FIG. 12, the FPs indicate potential new indications that have not been previously reported. Due to a relatively large number of FPs, when the candidates need to be narrowed down, the candidates can be narrowed down by calculating a decision function value of each indication in FPs and ranking each indication for each drug. FIG. 13 shows examples of decision function values of alendronate. Also, indications that are common to drugs already known to have similar action mechanisms (for example, alendronate and ricedronate, or aripiprazole and clozapine) and predicted to be FP are considered to have high potential as repositioned indications.

These results suggests that the prediction method of the present invention is also useful for drug repositioning.

Reference Signs List

**[0186]**

　　10/ training device
　　20/ prediction device
　　40/ server device
　　101/ processing part
　　201/ processing part
　　401/ processing part
　　400/ prediction system

105/ communication part
405/ communication part

## Claims

1. A method for training an artificial intelligence model, comprising:

   inputting a first training data set, a second training data set and a third training data set in association with one another into an artificial intelligence model to train the artificial intelligence model,
   the first training data set being a set of data in which a set of data indicating the dynamics of a biomarker in one organ or each of multiple different organs collected from respective non-human animals to which multiple predetermined existing substances with a known indication in humans have been individually administered is linked with labels indicating respective names of the administered predetermined existing substances,
   the second training data set being a set of data in which labels indicating respective names of the multiple predetermined existing substances are linked with labels indicating the indications reported for each of the multiple predetermined existing substances,
   the third training data set being a set of data in which labels indicating the indications reported for each of the multiple predetermined existing substances are linked with information about adverse events reported correspondingly to each of these indications,
   wherein the artificial intelligence model is for predicting an indication for a test substance in humans.

2. The training method according to Claim 1, wherein, in the training, the first training data set and the third training data set are linked by means of the second training data set to generate a fourth training data set, and the fourth training data set is input into the artificial intelligence model.

3. The training method according to Claim 1 or 2, wherein the information about adverse events includes labels indicating the adverse events, and the presence or absence or frequencies of occurrence of the adverse events in the indications.

4. The training method according to any one of Claims 1 to 3, wherein the biomarker is transcriptome.

5. The training method according to any one of Claims 1 to 4, wherein the artificial intelligence model is a One-Class SVM

6. A device for training an artificial intelligence model, comprising a processing part,

   wherein the processing part inputs a first training data set, a second training data set and a third training data set in association with one another into an artificial intelligence model to train the artificial intelligence model,
   the first training data set being a set of data in which a set of data indicating the dynamics of a biomarker in one organ or each of multiple different organs collected from respective non-human animals to which multiple predetermined existing substances with a known indication in humans have been individually administered is linked with labels indicating respective names of the administered predetermined existing substances,
   the second training data set being a set of data in which labels indicating respective names of the multiple predetermined existing substances are linked with labels indicating the indications reported for each of the multiple predetermined existing substances,
   the third training data set being a set of data in which labels indicating the indications reported for each of the multiple predetermined existing substances are linked with information about adverse events reported correspondingly to each of these indications, and
   wherein the artificial intelligence model is for predicting an indication for a test substance in humans.

7. A program for training an artificial intelligence model that, when executed by a computer, causes the computer to execute the step of inputting a first training data set, a second training data set and a third training data set in association with one another into an artificial intelligence model to train the artificial intelligence model,

   the first training data set being a set of data in which a set of data indicating the dynamics of a biomarker in one organ or each of multiple different organs collected from respective non-human animals to which multiple predetermined existing substances with a known indication in humans have been individually administered is linked with labels indicating respective names of the administered predetermined existing substances,
   the second training data set being a set of data in which labels indicating respective names of the multiple predetermined existing substances are linked with labels indicating the indications reported for each of the multiple predetermined existing substances,

the third training data set being a set of data in which labels indicating the indications reported for each of the multiple predetermined existing substances are linked with information about adverse events reported correspondingly to each of these indications,

wherein the artificial intelligence model is for predicting indication for a test substance in humans.

8. A method for predicting an indication for a test substance in humans, comprising the steps of:

acquiring a first test data set, the first test data set being a set of data indicating the dynamics of a biomarker in one or multiple organs collected from non-human animals to which a test substance has been administered, and

inputting the first test data set and a second test data set into an artificial intelligence model trained by a method according to any one of Claims 1 to 5 to use the trained artificial intelligence model to predict an indication for the test substance in humans based on the first test data set and the second test data set input thereinto, the second test data set being a set of data in which labels of multiple known indications are linked with information about adverse events reported correspondingly to each of the multiple known indications.

9. The prediction method according to Claim 7, wherein the test substance does not include an existing substance or an equivalent substance of an existing substance.

10. The prediction method according to Claim 7, wherein the test substance is one selected from existing substances or equivalent substances of existing substances.

11. A prediction device for predicting an indication for a test substance in humans, comprising a processing part,

wherein the processing part inputs a first test data set and a second test data set into an artificial intelligence model trained by a method according to any one of Claims 1 to 5 to use the trained artificial intelligence model to predict an indication for the test substance in humans based on the first test data set and the second test data set input thereinto,

the first test data set being a set of data indicating the dynamics of a biomarker in one or multiple organs corresponding to one or multiple organs collected from non-human animals to which the test substance has been administered to generate the first training data set,

the second test data set being a set of data in which labels of multiple known indications are linked with information, acquired to generate a third training data set, about adverse events reported correspondingly to each of the multiple known indications.

12. A computer program for predicting an indication for a test substance in humans that, when executed by a computer, causes the computer to execute the step of:

inputting a first test data set and a second test data set into an artificial intelligence model trained by a method according to any one of Claims 1 to 5 to use the trained artificial intelligence model to predict an indication for the test substance in humans based on the first test data set and the second test data set input thereinto,

the first test data set being a set of data indicating the dynamics of a biomarker in one or multiple organs corresponding to one or multiple organs collected from non-human animals to which the test substance has been administered to generate the first training data set,

the second test data set being a set of data in which labels of multiple known indications are linked with information, acquired to generate a third training data set, about adverse events reported correspondingly to each of the multiple known indications.

13. A prediction system for predicting an indication for a test substance in humans, comprising:

a server device for transmitting a first test data set, the first test data set being a set of data indicating the dynamics of a biomarker in one or multiple organs collected from non-human animals to which the test substance has been administered, and

a prediction device for predicting an action of the test substance on humans connected to the server device via a network,

the server device comprising a communication part for transmitting the first test data set,

the prediction device comprising a processing part and a communication part,

wherein the processing part acquires the first test data set transmitted via the communication part of the server device via the communication part of the prediction device, and

inputs the acquired first test data set and a second test data set into an artificial intelligence model trained by a method according to any one of Claims 1 to 5 to use the trained artificial intelligence model to predict an indication for the test substance in humans based on the first test data set and the second test data set input thereinto, the first test data set being a set of data indicating the dynamics of a biomarker in one or multiple organs collected from non-human animals to which the test substance has been administered to generate the first training data set,

the second test data set being a set of data in which labels of multiple known indications are linked with information, acquired to generate a third training data set, about adverse events reported correspondingly to each of the multiple known indications.

14. A method for using a first training data set, a second training data set and a third training data set to train an artificial intelligence model for predicting an indication for a test substance in humans,

the first training data set being a set of data in which a set of data indicating the dynamics of a biomarker in one organ or each of multiple different organs collected from respective non-human animals to which multiple predetermined existing substances with a known indication in humans have been individually administered is linked with labels indicating the names of existing substances administered to acquire the set of data indicating the dynamics of the biomarker, the second training data set being a set of data in which labels indicating respective names of the multiple predetermined existing substances are linked with labels indicating the indications reported for each of the multiple predetermined existing substances,

the third training data set being a set of data in which labels indicating the indications are linked with information about adverse events reported correspondingly to each of the indications.

15. A method for using a first test data set and a second test data set as test data for predicting an indication for a test substance in humans,

the first test data set being a set of data indicating the dynamics of a biomarker in one or multiple organs corresponding to one or multiple organs collected from non-human animals to which the test substance has been administered to generate the first training data set,

the second test data set being a set of data in which labels of multiple known indications are linked with information about adverse events reported correspondingly to each of the multiple known indications.

Fig.1

Fig.2

Approach of Patent Document 2

Data on the dynamics of
a biomarker in each of multiple
organs of animals in response
to the administration of
an existing substance

Actions of existing substances on humans.

Learn the data on the dynamics of
a biomarker in respective organs in
response to the administration of
an existing substance and actions
on humans.

Data on the dynamics of
a biomarker in each of
multiple organs in response
to the administration of
a test substance X.

Predict an action of the test substance X
on humans without relying on
mechanism prediction.

Fig.3

(A)

|  | Heart _Alas2 | Heart _Apod | Heart ... | Skin _Alas2 | Skin _Apod | Skin ... | ... |
|---|---|---|---|---|---|---|---|
| Aripiprazole | -0.2 | -0.39 | ... | -0.28 | -0.13 | ... | ... |
| EMPA | -0.1 | -0.04 | ... | 0.13 | 0.15 | ... | ... |
| ... | ... | ... | ... | ... | ... | ... | ... |

(B)

|  | Indication 1 | ... |
|---|---|---|
| Aripiprazole | Nerve injury | ... |
| EMPA | Type 2 diabetes mellitus | ... |
| ... | ... | ... |

(C)

|  | Sleep disorder | Blood glucose decreased | ... |
|---|---|---|---|
| Nerve injury | 0.026 | 0.009 | ... |
| Type 2 diabetes mellitus | 0.007 | 0.141 | ... |
| ... | ... | ... | ... |

(D)

| Sleep disorder | Blood glucose decreased | ... | Heart _Alas2 | Heart _Apod | Heart ... | Skin _Alas2 | Skin _Apod | Skin ... | ... |
|---|---|---|---|---|---|---|---|---|---|
| 0.026 | 0.009 | ... | -0.2 | -0.39 | ... | -0.28 | -0.13 | ... | ... |
| 0.007 | 0.141 | ... | -0.1 | -0.04 | ... | 0.13 | 0.15 | ... | ... |
| ... | ... | ... | ... | ... | ... | ... | ... | ... | ... |

Fig.4

(A)

50, 51

30                    10, 20

(B)

400

30                    10

Network

40                    20

Fig.5

| | |
|---|---|
| External database — 60 | |
| Measurement part — 30 | Communication I/F — 105 |
| Input part — 111 | Input I/F — 106 |
| Output part — 112 | Output I/F — 107 |
| Storage medium — 113 | Media I/F — 108 |

— 10

Processing part — 101

Main storage part — 102

ROM — 103

Auxiliary storage part — 104

OS — 1041

Training program — TP

Artificial intelligence model — AI1

First training data — TR1

Second training data — TR2

Third training data — TR3

109

Fig.6

```
                    ┌─────────────┐
                    │    Start    │
                    └──────┬──────┘
                           │
                           ▼                               S1
┌──────────────────────────────────────────────────────────┐
│  Acquire a first training data set, a second training data set  │
│              and a third training data set.                │
└──────────────────────────┬─────────────────────────────────┘
                           │
                           ▼                               S2
┌──────────────────────────────────────────────────────────┐
│             Generate a fourth training data set.           │
└──────────────────────────┬─────────────────────────────────┘
                           │
                           ▼                               S3
┌──────────────────────────────────────────────────────────┐
│        Input the fourth training data set into an artificial    │
│               intelligence model to train it.             │
└──────────────────────────┬─────────────────────────────────┘
                           │
                           ▼                               S4
┌──────────────────────────────────────────────────────────┐
│          Store the trained artificial intelligence model.  │
└──────────────────────────┬─────────────────────────────────┘
                           │
                           ▼
                    ┌─────────────┐
                    │     End     │
                    └─────────────┘
```

Fig.7

Fig.8

```
        ┌─────────┐
        │  Start  │
        └────┬────┘
             │
             ▼                                          S51
┌──────────────────────────────────────────────────────────┐
│    Acquire a first test data set and a second test data set.│
└──────────────────────────────────────────────────────────┘
             │
             ▼                                          S52
┌──────────────────────────────────────────────────────────┐
│    Input the first test data set and the second test data set│
│    into a trained artificial intelligence model to predict │
│          an indication for a test substance.               │
└──────────────────────────────────────────────────────────┘
             │
             ▼                                          S53
┌──────────────────────────────────────────────────────────┐
│                   Store the results.                       │
└──────────────────────────────────────────────────────────┘
             │
             ▼
        ┌─────────┐
        │   End   │
        └─────────┘
```

Fig.9

Fig.10

| Measurement part 30 | Server device 40 | Training Device 10 |
|---|---|---|

**Measurement part 30**

( Start )

S81
Acquire measurement values of a biomarker in each organ of non-human animals to which an existing substance has been administered.

S82
Transmit the measurement values.

S91
Acquire measurement values of a biomarker in each organ of non-human animals to which a test substance has been administered.

S92
Transmit the measurement values.

**Server device 40**

S83
Receive and store the measurement values.

S84
Transmit the measurement values.

S93
Receive the measurement values.

S94
Acquire the measurement values and generate test data.

S95
Acquire the artificial intelligence model.

S96
Use the artificial intelligence model to predict an action of the test substance on humans.

S97
Output the results.

**Prediction device 20**

**Training Device 10**

S85
Acquire information about indications for the existing substance and adverse events thereof.

S86
Acquire the measurement values.

S87
Generate a first training data set, second training data, and third training data.

S88
Generate fourth training data.

S89
Train and store an artificial intelligence model.

S90
Transmit the artificial intelligence model.

( End )

Fig.11

| Drug | TP | TN | FP | FN | Accuracy | Precision | Recall | F measure |
|---|---|---|---|---|---|---|---|---|
| Alendronate | 392 | 9249 | 1638 | 33 | | | | |
| APAP | 202 | 9879 | 0 | 1231 | | | | |
| Aripiprazole | 262 | 9495 | 1492 | 63 | | | | |
| Asenapine | 8 | 9211 | 2093 | 0 | | | | |
| Cisplatin | 0 | 10960 | 0 | 352 | | | | |
| Clozapine | 152 | 8952 | 2188 | 20 | | | | |
| Dox | 175 | 10635 | 345 | 157 | | | | |
| EMPA | 68 | 9146 | 2092 | 6 | | | | |
| Lenalidomide | 0 | 10903 | 0 | 409 | | | | |
| Lurasidone | 6 | 8918 | 2388 | 0 | | | | |
| Olanzapine | 260 | 9396 | 1599 | 57 | | | | |
| Repatha | 54 | 9362 | 1891 | 5 | | | | |
| Risedronate | 185 | 9281 | 1835 | 11 | | | | |
| Sofosbuvir | 107 | 9638 | 1556 | 11 | | | | |
| Teriparatide | 141 | 9162 | 2002 | 7 | | | | |

0.0      1.0  0.0      1.0  0.0      1.0  0.0      1.0

Fig.12

| Drug | TP | TN | FP | FN | Accuracy | Precision | Recall | F measure |
|---|---|---|---|---|---|---|---|---|
| Alendronate | 407 | 9016 | 1871 | 18 | | | | |
| APAP | 1298 | 8181 | 1698 | 135 | | | | |
| Aripiprazole | 295 | 9046 | 1941 | 30 | | | | |
| Asenapine | 8 | 9213 | 2091 | 0 | | | | |
| Cisplatin | 271 | 9470 | 1490 | 81 | | | | |
| Clozapine | 156 | 8809 | 2331 | 16 | | | | |
| Dox | 286 | 9311 | 1669 | 46 | | | | |
| EMPA | 70 | 9091 | 2147 | 4 | | | | |
| Lenalidomide | 362 | 9810 | 1093 | 47 | | | | |
| Lurasidone | 6 | 8919 | 2387 | 0 | | | | |
| Olanzapine | 283 | 8991 | 2004 | 34 | | | | |
| Repatha | 55 | 9306 | 1947 | 4 | | | | |
| Risedronate | 188 | 9198 | 1918 | 8 | | | | |
| Sofosbuvir | 109 | 9447 | 1747 | 9 | | | | |
| Teriparatide | 143 | 9083 | 2081 | 5 | | | | |

0.0     1.0  0.0     1.0  0.0     1.0  0.0     1.0

Fig.13

| Indication | Reported | Predicted | Confusion matrix terminology | Decision function value |
|---|---|---|---|---|
| Allergic sinusitis | 1 | 1 | TP | 8.98 |
| Antiviral prophylaxis | 1 | 1 | TP | 13.46 |
| Juvenile idiopathic arthritis | 1 | 1 | TP | 7.47 |
| Antiviral prophylaxis | 1 | 1 | TP | 13.46 |
| Urinary bladder sarcoma | -1 | -1 | TN | -22.30 |
| Encephalomyelitis | -1 | -1 | TN | -7.63 |
| Post procedural oedema | -1 | -1 | TN | -9.04 |
| Pneumonia cryptococcal | -1 | -1 | TN | -1.38 |
| Musculoskeletal disorder | -1 | 1 | FP | 11.34 |
| Chondrocalcinosis pyrophosphate | -1 | 1 | FP | 3.84 |
| Malignant nipple neoplasm | -1 | 1 | FP | 0.30 |
| T-cell prolymphocytic leukaemia | -1 | 1 | FP | 4.98 |
| Osteoporotic fracture | 1 | -1 | FN | -1.10 |
| Nephrogenic anaemia | 1 | -1 | FN | -7.67 |

## INTERNATIONAL SEARCH REPORT

| | International application No. |
|---|---|
| | PCT/JP2020/039179 |

### A. CLASSIFICATION OF SUBJECT MATTER
G16B 40/20(2019.01)i; A01K 67/027(2006.01)i; C12Q 1/6809(2018.01)i; G01N 33/15(2006.01)i; G01N 33/50(2006.01)i
FI: G16B40/20; A01K67/027; C12Q1/6809 Z; G01N33/15 Z; G01N33/50 P
According to International Patent Classification (IPC) or to both national classification and IPC

### B. FIELDS SEARCHED
Minimum documentation searched (classification system followed by classification symbols)
G16B5/00-99/00; G16C10/00-99/00; G16H10/00-80/00; G06Q10/00-99/00; A01K67/027; C12Q1/6809; G01N33/15; G01N33/50; G06N3/02

Documentation searched other than minimum documentation to the extent that such documents are included in the fields searched
| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 6559850 B1 (KARYDO THERAPEUTIX, INC.) 14 August 2019 (2019-08-14) paragraphs [0012]-[0016], [0022]-[0026], [0031], [0035]-[0040], [0043]-[0048], [0063]-[0064], [0082]-[0083] | 1-15 |
| A | JP 2019-502988 A (PREFERRED NETWORKS, INC.) 31 January 2019 (2019-01-31) paragraphs [0016], [0020], [0024]-[0028], [0064], [0069], [0078] | 1-15 |
| A | JP 2015-507470 A (COLD SPRING HARBOR LABORATORY, AN EDUCATION CORPORATION OF THE STATE OF NEW YORK) 12 March 2015 (2015-03-12) paragraphs [0021], [0027], [0032]-[0042], [0132] | 1-15 |
| A | US 2015/0371009 A1 (CHEN, Jake Yue) 24 December 2015 (2015-12-24) paragraphs [0050], [0054], [0066], [0070]-[0071], [0089], [0128] | 1-15 |

☐ Further documents are listed in the continuation of Box C. ☒ See patent family annex.

| | | |
|---|---|---|
| * | Special categories of cited documents: | |
| "A" | document defining the general state of the art which is not considered to be of particular relevance | "T" later document published after the international filing date or priority date and not in conflict with the application but cited to understand the principle or theory underlying the invention |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| 06 January 2021 (06.01.2021) | 19 January 2021 (19.01.2021) |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

International application No.

PCT/JP2020/039179

**INTERNATIONAL SEARCH REPORT**

Information on patent family members

| Patent Documents referred in the Report | Publication Date | Patent Family | Publication Date |
|---|---|---|---|
| JP 6559850 B1 | 14 Aug. 2019 | WO 2020/021857 A1 | |
| JP 2019-502988 A | 31 Jan. 2019 | US 2017/0161635 A1 paragraphs [0032], [0035], [0039]-[0043], [0076], [0082], [0092] WO 2017/094899 A1 | |
| JP 2015-507470 A | 12 Mar. 2015 | US 2014/0297199 A1 paragraphs [0072], [0078], [0083]-[0093], [0183] WO 2013/071099 A1 EP 2777074 A1 CN 104040719 A | |
| US 2015/0371009 A1 | 24 Dec. 2015 | (Family: none) | |

Form PCT/ISA/210 (patent family annex) (January 2015)

**REFERENCES CITED IN THE DESCRIPTION**

**Patent documents cited in the description**

- WO 2016208776 A
- JP 6559850 B